# EUROPÄISCHE PATENTSCHRIFT

(11) **EP 0 582 925 B1**
(45) Veröffentlichungstag und Bekanntmachung des Hinweises auf die Patenterteilung: **02.10.1996**
(21) Anmeldenummer: 93112327.7
(22) Anmeldetag: 31.07.1993
(51) Int. Cl.: C07C 251/48, A01N 37/50, C07C 255/62, A01N 37/34, C07D 261/08, C07D 317/60, C07D 319/18, C07D 271/107, C07D 213/54, C07D 333/24, C07D 277/66

(54) **Acetylenderivate und diese enthaltende Pflanzenschutzmittel**
Acetylenic derivatives and plant-protective agents containing them
Dérivés acétyléniques et des agents phytosanitaires les contenant

(30) Priorität: 11.08.1992 DE 4226557; 27.11.1992 DE 4239874
(43) Veröffentlichungstag der Anmeldung: 16.02.1994
(60) Teilanmeldung: 96101050.1
(73) Patentinhaber: BASF Aktiengesellschaft, 67063 Ludwigshafen (DE)
(72) Erfinder: Wingert, Horst, Dr., D-6800 Mannheim 1 (DE); Hellendahl, Beate, Dr., D-6707 Schifferstadt (DE); Kirstgen, Reinhard, Dr., D-6730 Neustadt (DE); Sauter, Hubert, Dr., D-6800 Mannheim 1 (DE); Ammermann, Eberhard, Dr., D-6148 Heppenheim (DE); Lorenz, Gisela, Dr., D-6730 Neustadt (DE)

(56) Entgegenhaltungen:
- EP-A- 0 178 826
- EP-A- 0 253 213
- EP-A- 0 280 185
- EP-A- 0 469 411
- EP-A- 0 477 631

## Beschreibung

Die vorliegende Erfindung betrifft neue Acetylenderivate und ein Verfahren zur Bekämpfung von Schädlingen, insbesondere von Pilzen, Insekten, Nematoden und Spinnmilben mit diesen Verbindungen.

Es ist bekannt, daß einzelne Acetylenderivate (EP-A 178 826, EP-A 244 077, EP-A 253 213, EP-A 280 185, EP-A 469 411, EP-A 477 631) fungizide oder insektizide Wirkung besitzen. Ihre Wirkung ist jedoch unbefriedigend.

Es wurde nun überraschend gefunden, daß die neuen Acetylenderivate der allgemeinen Formel I 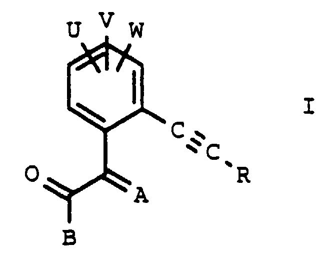 in der
- U,V,W: gleich oder verschieden sind und Wasserstoff, Halogen, Nitro, Cyano, C₁-C₄-Alkyl und C₁-C₄-Alkoxy bedeuten,

a) A C₁-C₄-Alkoxyimino oder C₁-C₄-Alkyliden und B C₁-C₄-Alkoxy bedeutet und
   - R: Wasserstoff, Halogen, Cyano, CF₃, ggf. verzweigtes C₁-F₁₀-Alkyl, Halogen-C₁-C₄-alkyl, C₁-C₄-Alkoxy-C₁-C₄-alkyl, C₂-C₆-Alkenyl, C₂-C₄-Alkinyl, C₃-C₆-Cycloalkyl, ggf. subst. Heterocyclyl, ggf. subst. Heteroaryl, ggf. subst. Aryl-C₁-C₄-alkyl, ggf. subst. Aryloxy-C₁-C₄-alkyl, ggf. subst. Arylamino-C₁-C₄-alkyl, ggf. subst. Arylthiomethyl, ggf. subst. Aryl-C₂-C₄-alkenyl, ggf. subst. Arylethinyl, ggf. subst. Heteroaryl-C₁-C₄-alkyl, ggf. subst. Heteroaryloxy-C₁-C₄-alkyl oder ggf. subst. Heteroarylthiomethyl bedeutet,
   COR¹, CONR²R³, CSNR⁴R⁵, COSR⁶, CSOR⁷, CSSR⁸, CH(OH)R⁹, CH(OR¹⁰)R¹¹, SiR¹²R¹³R¹⁴, SnR¹⁵R¹⁶R¹⁷, COR¹⁸ oder CR²⁰=NOR¹⁹ bedeutet, oder
   Aryl bedeutet, welches einen der folgenden Substituenten trägt: Cyano, C₁-C₄-Halogenalkoxy, C₁-C₄-Alkoxyimino-C₁-C₄-alkyl, Aryl, Aryloxy, Benzyl, Benzyloxy, Heteroaryl, Heteroaryloxy, C₃-C₆-Cycloalkyl, C₁-C₄-Dialkylamino, CO₂CH₃, C0₂C₂H₅, Formyl und Acyl, wobei der Ausdruck Heteroaryl einen aromatischen Fünfring- oder Sechsringheterocyclus bedeutet, oder
   Aryl bedeutet, welches mindestens zwei Substituenten aus der folgenden Gruppe trägt: Halogen, Cyano, Nitro, C₁-C₄-Alkyl, C₁-C₄-Alkoxy, C₁-C₄-Halogenalkyl, C₁-C₄-Halogenalkoxy, C₁-C₄-Alkoxyimino-C₁-C₄-alkyl, Aryl, Aryloxy, Benzyl, Benzyloxy, Heteroaryl, Heteroaryloxy, C₃-C₆-Cycloalkyl, C₁-C₄-Dialkylamino, CO₂CH₃, CO₂C₂H₅, Formyl und Acyl, wobei der Ausdruck Heteroaryl einen aromatischen Fünfring- oder Sechsringheterocyclus bedeutet, oder
b) A C₁-C₄-Alkoxyimino, C₁-C₄-Alkylthiomethyliden oder C₁-C₄-Alkoxymethyliden und B C₁-C₄-Alkylamino und
   - R: Wasserstoff, Halogen, Cyano, CF₃, Halogen-C₁-C₄-alkyl, C₁-C₄-Alkoxy-C₁-C₄-alkyl, C₂-C₆-Alkenyl, C₂-C₄-Alkinyl, C₃-C₆-Cycloalkyl, ggf. subst. Heterocyclyl, ggf. subst. Heteroaryl, ggf. subst. Aryl-C₁-C₄-alkyl, ggf. subst. Aryloxy-C₁-C₄-alkyl, ggf. subst. Arylamino-C₁-C₄-alkyl, ggf. subst. Arylthiomethyl, ggf. subst. Aryl-C₂-C₄-alkenyl, ggf. subst. Arylethinyl, ggf. subst. Heteroaryl-C₁-C₄-alkyl, ggf. subst. Heteroaryloxy-C₁-C₄-alkyl oder ggf. subst. Heteroarylthiomethyl bedeutet,
   COR¹, CONR²R³, CSNR⁴R⁵, COSR⁶, CSOR⁷, CSSR⁸, CH(OH)R⁹, CH(OR¹⁰)R¹¹, SiR¹²R¹³R¹⁴, SnR¹⁵R¹⁶R¹⁷, COR¹⁸ oder CR²⁰=NOR¹⁹ bedeutet, oder
   Aryl bedeutet, welches mindestens einen Substituenten aus der folgenden Gruppe trägt: Halogen, Cyano, Nitro, C₁-C₄-Alkyl, C₁-C₄-Alkoxy, C₁-C₄-Halogenalkyl, C₁-C₄-Halogenalkoxy, C₁-C₄-Alkoxyimino-C₁-C₄-alkyl, Aryl, Aryloxy, Benzyl, Benzyloxy, Heteroaryl, Heteroaryloxy, C₃-C₆-Cycloalkyl, C₁-C₄-Dialkylamino, CO₂CH₃, CO₂C₂H₅, Formyl und Acyl, wobei der Ausdruck Heteroaryl einen aromatischen Fünfring- oder Sechsringheterocyclus bedeutet, oder
c) A C₁-C₄-Alkyliden und B C₁-C₄-Alkylamino oder
   A C₁-C₄-Alkylthiomethyliden und B C₁-C₄-Alkoxy oder
   A C₁-C₄-Alkyliden, C₁-C₄-Alkoxymethyliden, C₁-C₄-Alkylthiomethyliden und C₁-C₄-Alkoxyimino und B Hydroxy bedeutet und
   - R: Wasserstoff, Halogen, Cyano, CF₃, ggf. verzweigtes C₁-C₁₀-Alkyl, Halogen-C₁-C₄-alkyl, C₁-C₄-Alkoxy-C₁-C₄-alkyl, C₂-C₆-Alkenyl, C₂-C₄-Alkinyl, C₃-C₆-Cycloalkyl, ggf. subst. Heterocyclyl, ggf. subst. Aryl, ggf. subst. Heteroaryl, ggf. subst. Aryl-C₁-C₄-alkyl, ggf. subst. Aryloxy-C₁-C₄-alkyl, ggf. subst. Arylamino-C₁-C₄-alkyl, ggf. subst. Arylthiomethyl, ggf. subst. Aryl-C₂-C₄-alkenyl, ggf. subst. Arylethinyl, ggf. subst. Heteroaryl-C₁-C₄-alkyl, ggf. subst. Heteroaryloxy-C₁-C₄-alkyl oder ggf. subst. Heteroarylthiomethyl bedeutet,
   COR¹, CONR²R³, CSNR⁴R⁵, COSR⁶, CSOR⁷, CSSR⁸, CH(OH)R⁹, CH(OR¹⁰)R¹¹, SiR¹²R¹³R¹⁴, SnR¹⁵R¹⁶R¹⁷, COR¹⁸ oder CR²⁰=NOR¹⁹ bedeutet,
   - R¹-R¹⁷, R¹⁹ und R²⁰: gleich oder verschieden sind und
   Wasserstoff, C₁-C₄-Alkyl, ggf. subst. Aryl, ggf. subst. Heteroaryl, ggf. subst. Arylalkyl und ggf. subst. Heteroarylalkyl bedeuten,
   - R¹⁸: OH, C₁-C₄-Alkoxy und ggf. subst. Aryl-C₁-C₄-alkoxy bedeutet
   wobei der Ausdruck "ggf. subst." neben Wasserstoff für die Substituenten Halogen, Cyano, Nitro, C₁-C₄-Alkyl, C₁-C₄-Alkoxy, C₁-C₄-Halogenalkyl, C₁-C₄-Halogenalkoxy, C₁-C₄-Alkoxyimino-C₁-C₄-alkyl, Aryl, Aryloxy, Benzyl, Benzyloxy, Heteroaryl, Heteroaryloxy, C₃-C₆-Cycloalkyl, C₁-C₄-Dialkylamino, CO₂CH₃, CO₂C₂H₅, Formyl und Acyl steht und der Ausdruck Heteroaryl einen aromatischen Fünfring- oder Sechsringheterocyclus bedeutet,
   eine ausgezeichnete fungizide, insektizide, nematizide und akarizide Wirkung haben.

Die fungizide und insektizide Wirkung wird bevorzugt.

Die in der allgemeinen Formel I aufgeführten Reste können beispielsweise die folgende Bedeutung haben:
- U, V, W: können Wasserstoff, Halogen (z. B. Fluor, Chlor, Brom, Jod), Methyl, Methoxy bedeuten,
- A: kann C₁-C₄-Alkyliden (z. B. Methyliden, Ethyliden, n-oder iso-Propyliden, n-, iso-, sec.- oder tert.-Butyliden), C₁-C₄-Alkoxymethyliden (z. B. Methoxy-, Ethoxy-, n- oder iso-Propoxy-, n-, iso-, sec.- oder tert.-Butoxymethyliden), C₁-C₄-Alkylthiomethyliden (z. B. Methyl-, Ethyl-, n- oder iso-Propyl-, n-, iso-, sec.- oder tert.-Butylthiomethyliden), C₁-C₄-Alkoxyimino (z. B. Methoxy-, Ethoxy-, n- oder iso-Propoxy-, n-, iso-, sec.- oder tert.-Butoxyimino)
- B: kann OH, C₁-C₄-Alkoxy (z. B. Methoxy, Ethoxy, n- oder iso-Propoxy, n-, iso-, sec.- oder tert.-Butoxy) und C₁-C₄-Alkylamin (z. B. Methylamin, Ethylamin, n- oder iso-Propylamin, n-, iso-, sec.- oder tert.-Butylamin) bedeuten und
- R: Wasserstoff, Halogen (Fluor, Chlor, Brom, Jod), Cyano, CF₃, ggf. verzweigtes C₁-C₁₀-Alkyl (z. B. Methyl, Ethyl, n- oder iso-Propyl, n-, iso-, sec.- oder tert.-Butyl, n-, iso-, sec.-, tert.- oder neo-Pentyl, n-Hexyl, n-Decyl), C₃-C₆-Cycloalkyl (z. B. Cyclopropyl, Cyclobutyl, Cyclopentyl, Cyclohexyl), ggf. subst. Aryl (z. B. Phenyl, Naphthyl, Anthryl), Halogen-C₁-C₄-alkyl (z.B. Brommethyl, Chlormethyl, Jodmethyl, CF₃-methyl), C₂-C₆-Alkenyl (z. B. Vinyl, 1-Propenyl, 2-Propenyl, 2-Butenyl, 3-Butenyl, 1-Methyl-2-propenyl, 3-Methyl-2-butenyl, 2-Methyl-2-penten-5-yl), C₂-C₄-Alkinyl (z. B. Ethinyl, 1-Propinyl, ggf. subst. Heterocyclyl (z. B. Oxiranyl, 1-Aziridinyl, 1-Azetidinyl, 1-Pyrrolidinyl, 2-Tetrahydrofuryl, 2-Tetrahydropyranyl, 3-Tetrahydropyranyl, 1-Piperidinyl, 1-Morpholinyl, 1-Piperazinyl, 1,3-Dioxanyl, 3-Tetrahydrothiopyranyl), ggf. subst. Heteroaryl (z. B. Pyridyl, 2-Pyridyl, 3-Pyridyl, 4-Pyridyl, Pyrimidinyl, 4-Pyrimidinyl, 2-Pyrimidinyl, Thienyl, 2-Thienyl, 3-Thienyl, Furyl, 2-Furyl, 3-Furyl, 1-Pyrrolyl, 5-Isoxazolyl, 3-Isoxazolyl, 2-(1,3,4-Oxadiazol)yl, 5-(1,2,4-Oxadiazol)yl, 2-(1,3,4-Thiadiazol)yl, 1-Imidazolyl, 1,2,4-Triazolyl, 1,3,4-Triazolyl, 4-Thiazolyl, 2-Benzothiazolyl), ggf. subst. Aryl-C₂-C₄-alkyl (z. B. Benzyl, 1-Phenethyl, 2-Phenethyl, 1-Phenylpropyl, 2-Phenylpropyl, 3-Phenylpropyl, 2-Methyl-3-phenylpropyl, 2-Methyl-2-phenylpropyl, 4-Phenylbutyl), ggf. subst. Aryl-C₁-C₄-alkenyl (z. B. Phenyl-1-ethenyl, 2-Phenyl-1-propenyl, 2,2-Diphenylethenyl, 1-Phenyl-1-propen-2-yl, 1-Phenyl-1-propen-2-yl, ggf. subst. Arylethinyl (z. B. Phenylethinyl), ggf. subst. Heteroaryl-C₁-C₄-alkyl (z. B. Pyridylmethyl, 3-Pyridylmethyl), C₁-C₄-Alkoxy-C₁-C₄-alkyl (z. B. Methoxymethyl, Ethoxymethyl, n- oder iso-Propoxymethyl, n-, iso-, sec.- oder tert.-Butoxymethyl, 2-Methoxy-prop-2-yl, 2-Ethoxyprop-2-yl, 2-n-oder iso-Propxyprop-2-yl, 2-n-, iso-, sec.- oder tert.-Butoxyprop-2-yl), ggf. subst. Aryloxy-C₁-C₄-alkyl (z. B. Phenoxymethyl), ggf. subst. Heteroaryloxy-C₁-C₄-alkyl (z. B. 2-Thienyloxymethyl), ggf. subst. Arylamino-C₁-C₄-alkyl (z. B. N-(Phenylamino)-methyl), C(O)R¹ (z. B. C(O)CH₃, C(O)CH₂CH₃, C(O)Phenyl), C(O)NR²R³ (z. B. C(O)NH₂, C(O)NHCH₃), C(S)NR²R³ (z. B. C(S)NH₂, C(S)NHCH₃), C(O)SR⁶ (z. B. C(O)SCH₃, C(O)SCH₂C₆H₅), C(O)OR⁷ (z. B. CO₂CH₃, CO₂CH₂C₆H₅), C(S)SR⁸ (z. B. CS₂CH₃), CH(OH)R⁹ (z. B. H(OH)C₆H₅), CH(OR¹⁰)R¹¹ (z. B. CH(OCH₃)C₆H₅), SiR¹²R¹³R¹⁴ (z. B. SiMe₃), SnR¹⁵R¹⁶R¹⁷ (z. B. SnMe₃), C(O)R¹⁸ (z.B. CO₂Me, CO₂CH₂C₆H₅), C(=N-OR¹⁹)R²⁰ (z.B. C(=NOMe)C₆H₅).
- R¹-R¹⁷, R¹⁹, R²⁰: bedeuten beispielsweise Wasserstoff, C₁-C₄-Alkyl (z.B. Methyl, Ethyl, n-, iso-Propyl, n-, iso-, sec- und n-Butyl), ggf. subst. Aryl (z.B. 2-Chlorphenyl, 3,5-Dimethylphenyl, 1-Methyl-2-naphthyl), ggf. subst. Hetaryl (z.B. 6-Cl-2-pyrridyl, 4-pyrimidinyl, 2-Furyl, 6-Methyl-benzthiazol-2-yl), ggf. subst. Arylalkyl (z.B. 2-Chlorbenzyl, 3,5-Dichlorbenzyl) und ggf. subst. Heteroarylalkyl (z.B. 2-Furylmethyl, 6-Methyl-2-pyridylmethyl) und
- R¹⁸: bedeutet beispielsweise OH, C₁-C₄-Alkoxy (z.B. Methoxy, Ethoxy), ggf. subst. Aryl-C₁-C₄-Alkoxy (z.B. 2-Chlorbenzyloxy).

Die mit "ggf. subst." bezeichneten Reste im Vorstehenden enthalten neben Wasserstoff z. B. als Substituenten Fluor, Chlor, Brom, Jod, Cyano, Nitro, Methyl, Ethyl, iso-Propyl, tert.-Butyl, Methoxy, Ethoxy, iso-Propoxy, tert.-Butoxy, Trifluormethyl, Chlormethyl, Dichlormethyl, Trichlormethyl, Trifluormethoxy, 1,1,2,2-Tetrafluorethoxy, Methoxyiminomethyl, Ethoxyiminomethyl, n-Propoxyiminomethyl, n-Butoxyiminomethyl, n-Pentoxyiminomethyl, n-Hexoxoyiminomethyl, Allyloxyiminomethyl, Benzyloxyiminomethyl, iso-Propoxyiminomethyl, iso-Butoxyiminomethyl, tert.-Butoxyiminomethyl, Methylimino-1-ethyl, Ethoxyimino-1-ethyl, n-Propoxyimino-1-ethyl, n-Butoxyimino-1-ethyl, n-Pentoxyimino-1-ethyl, n-Hexoxyimino-1-ethyl, Allyloximini-1-ethyl, Benzyloxyimino-1-ethyl, Phenyl, Phenoxy, Benzyl, Benzyloxy, Imidazol-1-yl, Piperazin-1-yl, 4-Morpholinyl, Piperidin-1-yl, Pyridyl-2-oxy, Cyclopropyl, Cyclohexyl, Oxiranyl, 1,3-Dioxan-2-yl, 1,3-Dioxolan-2-yl, Tetrahydropyran-2-yloxy, Dimethylamino, Diethylamino, CO₂Me, CO₂Et, Formyl und Acyl, wobei Me Methyl und Et Ethyl bedeutet.

Bevorzugt sind Verbindungen der allgemeinen Formel I, 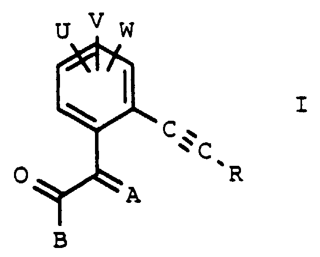 in der
- U, V und W Wasserstoff bedeuten, A für Methoxyimino und B für Methoxy steht;
- U, V und W Wasserstoff bedeuten, A für Ethyliden und B für Methoxy steht;
- U, V und W Wasserstoff bedeuten, A für Methoxyimino und B für Methylamino steht;
- U, V und W Wasserstoff bedeuten, A für Methylthiomethyliden und B für Methoxy steht;
- U, V, W und R für Wasserstoff stehen.

Dieneuen Verbindungen der allgemeinen Formel I können bei der Herstellung aufgrund der C=C- bzw. C=N-Doppelbindungen als E/Z-Isomerengemische anfallen. Diese können in üblicher Weise, z.B. durch Kristallisation oder Chromatographie in die einzelnen Komponenten aufgetrennt werden. Sowohl die einzelnen isomeren Verbindungen als auch ihre Gemische werden von der Erfindung umfaßt und sind als Schädlingsbekämpfungsmittel brauchbar.

Die Herstellung der Verbindungen der allgemeinen Formel I erfolgt beispielsweise wie in den folgenden Schemata beschrieben.

Die neuen Verbindungen der allgemeinen Formel I können hergestellt werden, indem man beispielsweise eine o-Brom-Verbindung 2 mit einem terminalen Acetylenderivat 3 in Gegenwart eines Übergangsmetallkatalysators, vorzugsweise in Gegenwart einer Palladiumverbindung, wie z.B. Pd(PPh₃)₄, PdCl₂, Pd(OAc)₂, in Gegenwart von Triphenylphosphin und in Gegenwart einer Base, wie z.B. in Gegenwart eines N-Alkylamins, wie beispielsweise Triethylamin, ggf. in Gegenwart eines Lösungs- oder Verdünnungsmittels, wie z.B. DMF oder Acetonitril umsetzt [vgl. z.B. L. Brandsma et al., Synth. Comm. 20, 1889 (1990); W. B. Austin et al., J. Org. Chem. 46, 2280 (1981)]. 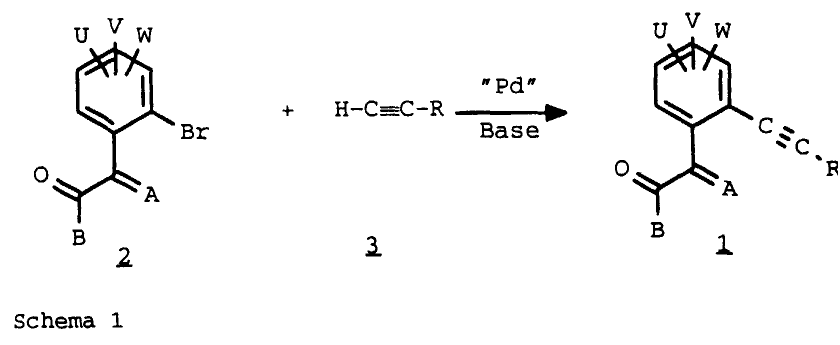 A, B, R, U, V und W haben die in Anspruch 1 angegebene Bedeutung.

Alternativ hierzu erhält man die Verbindungen der allgemeinen Formel 1, indem man die o-Brom-α-Ketosäurederivate 4 mit den Acetylenen der Formel 3 umsetzt (Bedingungen analog Schema 1) 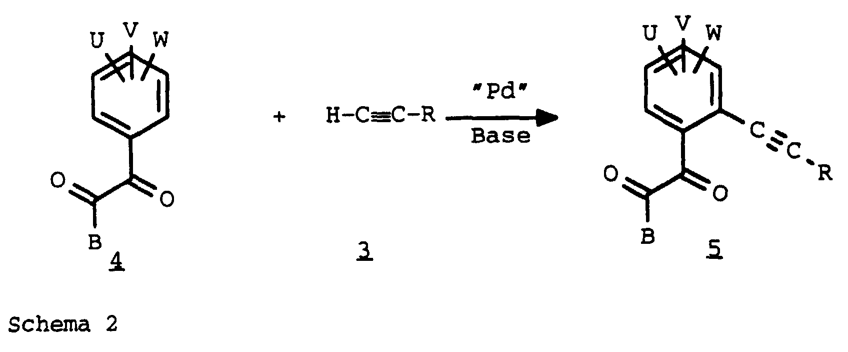 und anschließend die erhaltenen α-Ketocarbonsäurederivate 5
a) zum Beispiel im Sinne einer Wittig- bzw. im Sinne einer Wittig-Horner-Emmons-Reaktion mit einem Phosphoniumylid 6 bzw. mit einem Phosphonsaurederivat 7 (Schema 3) zu den neuen Verbindungen der Formel 1 umsetzt; wobei B, U, V, W und R die in Anspruch 1 angegebene Bedeutung haben und in der A: CH-Alkyl, CH-Alkoxy und CH-Thioalkyl bedeutet (vgl. z. B. C. Ferri; Reaktionen der organischen Synthese, Thieme Verlag, Stuttgart, S. 354 ff, 1978), oder 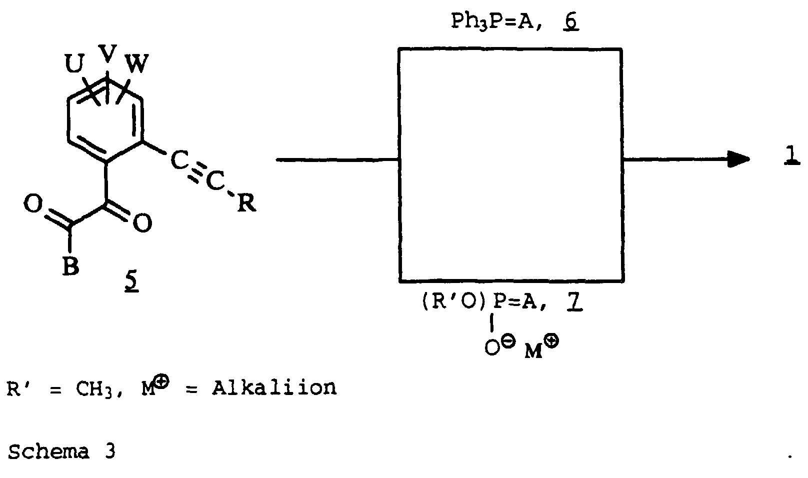
b) mit einem O-Alkylhydroxylamin 8, gegebenenfalls in Gegenwart einer Säure, z. B. Salzsäure, oder einer Base, z. B. K₂CO₃ zu den neuen Verbindungen der Formel 1 umsetzt, in der B, U, V, W, R und R⁴ die in Anspruch 1 gegebene Bedeutung haben (vgl. z. B. EP 253213).

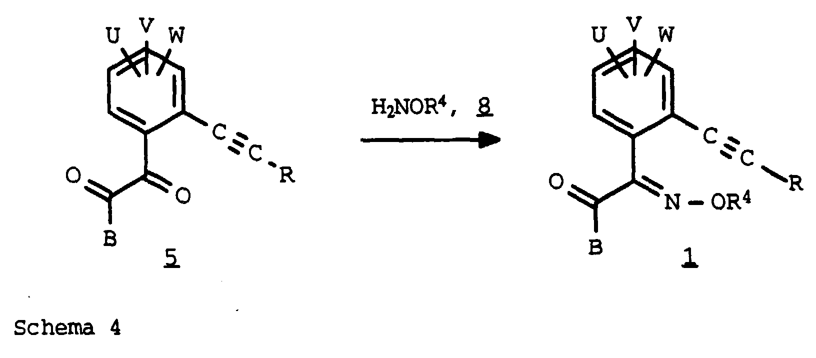

Die neuen Verbindungen der allgemeinen Formel 1 lassen sich auch herstellen, indem man die terminalen Acetylene 9 im Sinne einer Übergangsmetall(ÜM)-katalysierten Kupplung mit den Halogeniden 10, vorzugsweise mit den Bromiden (X = Br) bzw. den Jodiden (X = J) in Gegenwart eines Katalysators, wie z. B. PdCl₂, Pd(OAc)₂ oder Pd(PPh₃)₄ in Gegenwart einer Base, wie etwa Triethylamin zu Reaktion bringt (vgl. z. B. P.C. Vollhardt, Angew. Chem. 98, 268 (1986), H. Yamanaka, Synthesis, 312 (1983)).

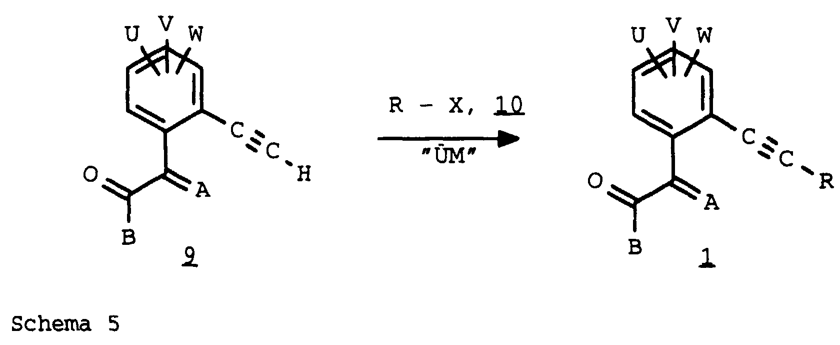

Die Reste A, B, R, U, V und W haben die in Anspruch 1 gegebene Bedeutung.

Alternativ hierzu kann man auch so vorgehen, daß man die α-Ketosäurederivate der terminalen Acetylene 11 mit den Halogeniden 10 übergangsmetall(ÜM)katalysiert in die Derivate 5 Überführt und diese wie zuvor beschrieben in die neuen Verbindungen der Formel 1 überführt.

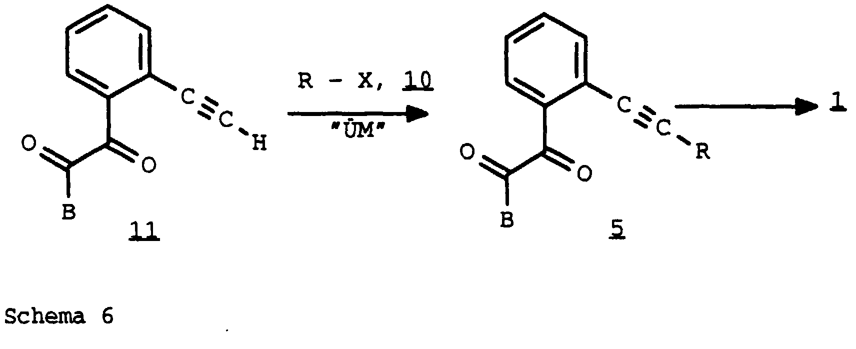

Eine weitere Möglichkeit zur Darstellung der Verbindungen der allgemeinen Formel 1 besteht darin, daß man zuerst die Acetylengruppierung in Formel 12, nach bekannten Methoden (vgl. z. B. Houben-Weyl, Bd. V/2a, S. 33 f Viehe, bzw. siehe Schema 1 + 2) aufbaut, 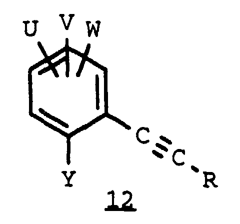 und anschließend die Gruppe Y in die Gruppierung 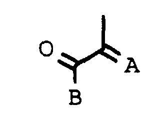 überführt.

Schemata 7-9 sollen dies verdeutlichen. Die Ketocarbonsäureester 17 lassen sich ausgehend von den o-Brombenzaldehyden 13 darstellen.

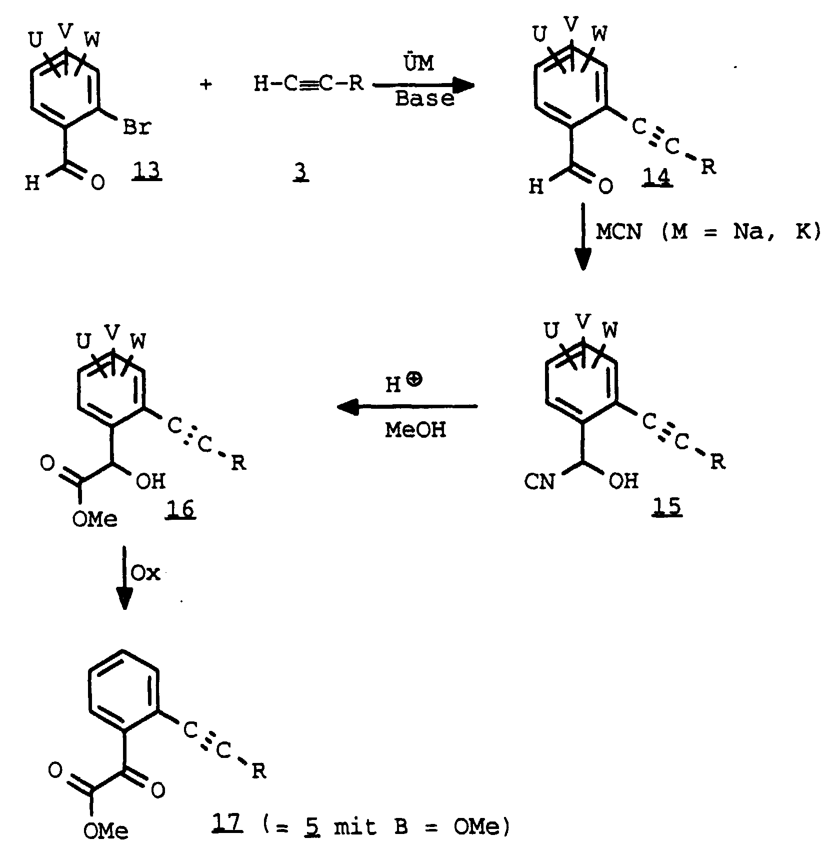

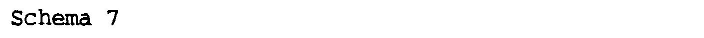

Die o-Brom-benzaldehyde 12 lassen sich mit den terminalen Acetylenen 3 Übergangsmetall-katalysiert zu den Aldehyden 14 umsetzen (vgl. W.B. Austin, J. Org. Chem., 46 2280, (1981)).

Aus den Aldehyden 14 erhält man die Cyanhydrine 15 durch Umsetzung mit einem Metallcyanid, wie NaCN oder KCN in Gegenwart einer Säure, z. B. Salzsäure (vgl. z. B. Organikum, 16. Auflage, S. 445 (1986)).

Über eine "Pinner-Reaktion" lassen sich aus den Cyanhydrinen 15 die Mandelsäurederivate 16 darstellen (vgl. z.B. Org. Synth. Coll. Vol. 4, 58 (1963)).

Die Oxidation der Mandelsäurederivate 16, beispielsweise mit Chromverbindungen, wie Chromtrioxid, mit Natriumhypochlorid oder mit TEMPO liefert die α-Ketoester 17 (vgl. z. B. Houben-Weyl, Bd. 4/1b, S. 425-464 (1975) (TEMPO = Tetramethylpiperidin-l-oxyl).

Aus den Ketoestern 17 lassen sich die neuen Verbindungen der Formel 1a, in denen A, U, V, W und R die in Anspruch 1 beschriebene Bedeutung haben und B für die Gruppierung CO₂Me steht, wie in den Schemata 3 und 4 aufgezeigt, herstellen.

Die α-Ketocarbonsäureester 17 lassen sich auch ausgehend von den Halogenderivaten 18 durch Grignard-Reaktion mit Oxalsäureverbindungen 19 analog Schema 8 aufbauen (vgl. z. B. J.S. Nimitz, J. Org. Chem., 46, 211 (1981).

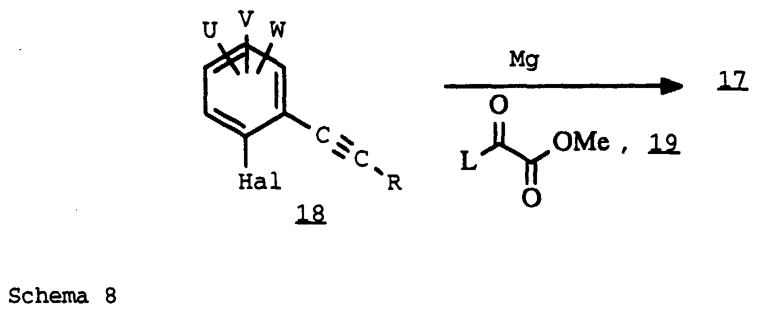

Eine weitere Möglichkeit besteht darin, Carbonsäuren 20 über die Stufe der Säurechloride in die Cyanide 21 zu Überführen und diese im Sinne einer Pinnerreaktion zu den Ketoestern 17 umzuwandeln. 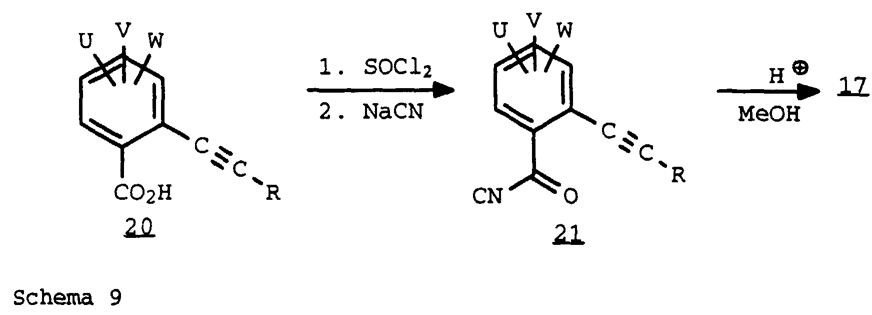 (vgl. z. B. Organikum, 16. Auflage, 423 f (1986), Angew. Chem. 94, 1, 1982)).

Aus den Carbonsäureesterderivaten 1a können die neuen Verbindungen der Formel 1, in der A, X, Y, Z und R die in Anspruch 1 gegebene Bedeutung haben und B Hydroxy oder Alkylamino bedeutet, hergestellt werden.

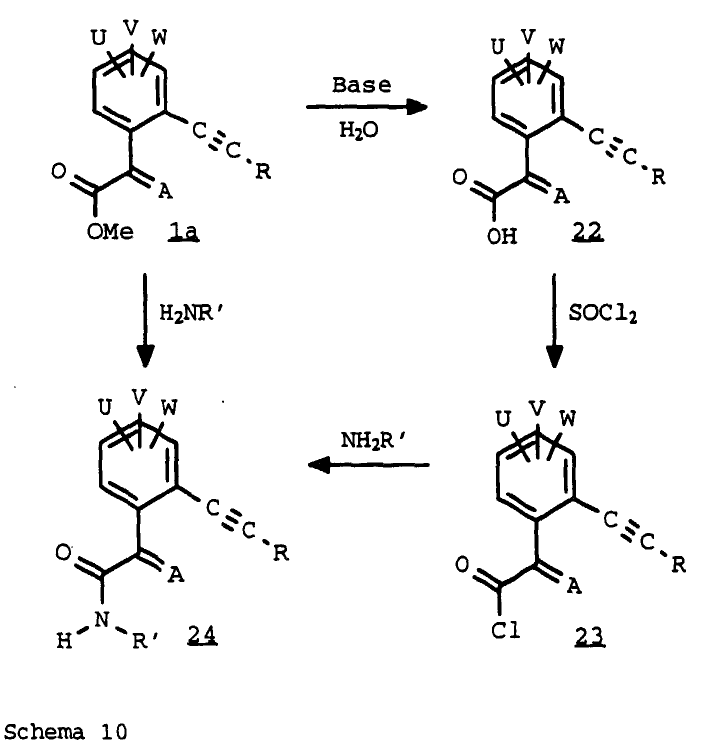

Durch Verseifung erhält man die Carbonsäuren 22 nach bekannten Verfahren (vgl. Organikum, 16. Auflage, S. 415 f, S. 622 (1986)).

Diese lassen sich in die Säurechloride 23 überführen (vgl. Organikum, 16. Auflage, S. 423 f (1986)).

Die Amide 24 erhält man entweder hieraus auf an sich bekannte Weise (Organikum, 16. Auflage, S. 412 (1986)), oder alternativ hierzu aus den Carbonsäureestern 1a direkt durch Aminolyse mit Aminen.

Die neuen Verbindungen der allgemeinen Formel I lassen sich aber auch so darstellen, daß man eine geeignet substituierte Styryl-Verbindung 25 in an sich bekannter Weise in das entsprechende Acetylenderivat 1 überführt.

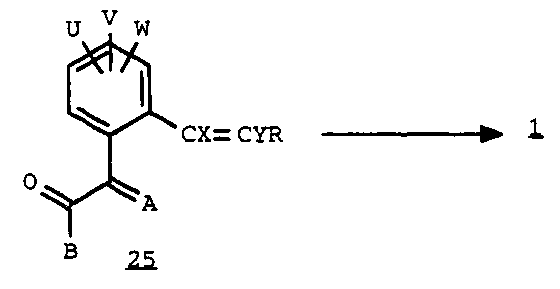

Für den Fall, daß X = H und Y = Halogen bzw. X = Halogen und Y = H, wird die Reaktion in Gegenwart einer Base wie z. B. Natriumhydrid, Kaliumhydroxid, Triton B oder n-Butyllithium durchgeführt (vgl. z. B. H. Zimmer et al, Chimia 28 (1974), S. 656 f) (Triton B = 2-Benzyltrimethylammoniumhydroxid).

Im Falle von X und Y = Halogen läßt sich die Abspaltung mit Zink durchführen (vgl. z. B. H.G. Viehe, Chemistry of Acetylenes, Marcel Dekker, New York, 1969, S. 134 f).

Die folgenden Beispiele erläutern die Herstellung der Verbindungen.

### Beispiele

### Beispiel 1

### Darstellung von 2-Ethinylphenylglyoxylsäure-methylester-O-methyloxim (Verbindung 1, Tabelle 2)

Zu einer Lösung von 55,4 g (0,23 mol) 2-Bromphenylglyoxylsäuremethylester in 415 ml Triethylamin gibt man 33,3 g (0,35 mol) Trimethylsilylacetylen, 3,8 g Palladium(II)acetat, 3,2 g Kupfer(I)jodid und 8,9 g Triphenylphosphin und leitet anschließend 30 min Stickstoff durch die Lösung. Das Reaktionsgemisch wird anschließend 45 min auf 90°C erhitzt. Man läßt abkühlen und filtriert ab. Das Filtrat wird eingeengt, in Essigester aufgenommen und mit Wasser gewaschen. Die organische Phase wird getrocknet und eingeengt. Es verbleiben 56,8 g α-Ketoester als schwarzes Öl.

Das auf diese Weise dargestellte Rohprodukt wird in 50 ml Methanol gelöst, mit 38,9 g (0,37 mol) O-Methylhydroxylaminhydrochlorid versetzt und 15 min auf 60 °C erhitzt. Man engt ein, nimmt den Rückstand in Essigester auf und wäscht mit Wasser. Die organische Phase wird getrocknet und eingeengt. Es verbleiben 52,4 g der Verbindung Nr. 2, Tabelle 2 als schwarzes Öl. ¹H-NMR (CDCl₃/TMS): δ = 0,22 (s, 9H, SiMe₃); 3,86; 4,06 (s, 3H, OCH₃); 7,25-7,61 ppm (m, 4H, Aryl).

Die auf diese Weise dargestellte Trimethylsilylacetylen-Verbindung wird in 320 ml Methanol gelöst und zusammen mit 3,2 g Kaliumcarbonat 1 Stunde bei Raumtemperatur (20°C) gerührt. Man engt anschließend ein, nimmt den Rückstand in Methylenchlorid auf und wäscht mit 10%iger Natriumhydrogencarbonat-Lösung. Die organische Phase wird getrocknet und eingeengt. Es verbleiben 36 g (72 % bezogen auf 2-Brom-phenylglyoxylsäuremethylester) der Verbindung 1, Tabelle 2 als schwarzer Feststoff. ¹H-NMR (CDCl₃/TMS): δ = 3,17 (s, 3H, ≡C-H); 3,87; 4,07 (s, 3H, OCH₃); 7,27 - 7,60 ppm (m, 4H, Aryl).

### Beispiel 2

### a) Darstellung von 2 (2-Methylphenyl)ethinylphenylglyoxylsäuremethyl-ester-O-methyloxim (EP 253213, Nr. 136)

Zu einer Lösung von 10 g (0,041 mol) 2-Bromphenylglyoxylsäuremethylester in 50 ml Triethylamin gibt man 300 mg Palladium(II)acetat, 1,5 g Triphenylphosphin und 100 mg Kupfer(I)jodid und 11 g (0,095 mol) 2-Methylphenylacetylen. Nachdem man 30 min Stickstoff durch die Lösung geleitet hat, erhitzt man 3 Stunden auf 80°C. Nach dem Abkühlen gibt man Methylenchlorid zu und wäscht mit Wasser. Die organische Phase wird getrocknet und eingeengt. Es verbleiben 16,5 g 2-(2-Methylphenyl)ethinylphenylglyoxylsäuremethylester als schwarzes Öl. ¹H-NMR (CDCl₃/TMS): δ = 2,53 (s, 3H, CH₃); 3,81 (s, 3H, OCH₃); 7,13 -, 7,88 ppm (m, 8H, Aryl).

Der auf diese Weise erhaltene Ketoester wird in 20 ml Methanol mit 7,4g (0,089 mol) Methoxyaminhydrochlorid versetzt und 1,5 Stunden unter Rückfluß erhitzt. Man engt ein, nimmt den Rückstand in Essigester auf, wäscht mit Wasser, trocknet die organische Phase und engt ein. Es verbleiben 9,5 g Produkt als schwarzes Öl, das aus Methanol umkristallisiert werden kann. Man erhält auf diese Weise die o. a. Verbindung als farblosen Feststoff mit einem Schmelzpunkt von 92 - 96°C. ¹H-NMR (CDCl₃/TMS) δ = 2,46 (s, 3H, CH₃); 3,83; 4,07 (s, 3H, OCH₃); 7,12 - 7,63 ppm (m, 8H, Aryl).

### b) 2-(2-Methylphenylethinyl)phenylglyoxylsäure-N-methylamid-O-methyloxim (Verbindung 32, Tabelle 4)

Zu 50 ml einer 40 %igen Methylaminlösung gibt man 2 g (7 mmol) der in Beispiel 2a dargestellten Verbindung und rührt Über Nacht bei Raumtemperatur. Anschließend wird mit Ether extrahiert, getrocknet und eingeengt. Es verbleiben 0,9 g (42 %) der Verbindung 32 Tabelle 4 als hellgelber Feststoff. m.p. 128 - 129°C. ¹H-NMR (CDCl₃/TMS) δ = 2,43 (s, 3H, CH₃); 2,91 (d, 3H, NCH3); 3,96 (s, 3H, OCH₃); 6,75 (br, 1H, NH); 7,14 -, 7,63 ppm (m, 8H, Aryl).

### Beispiel 3

### 2(2-(3-Chlor)thienylethinyl)phenylglyoxylsäuremethylester-O-methyloxim (Verbindung 178, Tabelle 2)

Zu einer Lösung von 2 g (0,009 mol) 2-Ethinylphenylglyoxylsäuremethylester-O-methyloxim (Beispiel 1) und 3,6 g (0,018 mol) 2-Brom-,3-Chlorthiophen in 100 ml Triethylamin gibt man 100 mg Palladium(II)acetat, 87 mg Kupfer(I)jodid und 240 mg Triphenylphosphin. Man leitet 30 min Stickstoff durch die Lösung und erhitzt anschließend 1 Stunde auf 90°C. Der entstandene Feststoff wird abfiltriert und das Filtrat wird eingeengt. Man nimmt den Rückstand in Essigester auf, wäscht mit Wasser, trocknet die organische Phase und engt ein.

Das verbleibende Rohprodukt wird an Kieselgel mit Hexan/Methyl-tert.-butylether chromatographiert.

Man erhalt als 1. Fraktion 700 mg (23 %) Verbindung 178, Tabelle 2 als braunes Öl. ¹H-NMR (CDCl₃/TMS): δ = 3,88; 4,09 (s, 3H, OCH₃); 6,93 (d, 1H, =CH); 7,25 (d, 1H, =CH); 7,27 - 7,66 ppm (m, 4H, Aryl).

Als 2. Fraktion erhält man 500 mg (13 %) "dimeres" Ausgangsmaterial (Verbindung 162, Tabelle 2). ¹H-NMR (CDCl₃/TMS): δ = 3,91; 4,09 (s, 3H, OCH3); 7,27 - 7,61 ppm (m, 4H, Aryl).

### Vorschrift 1

### α-[2-(Phenylethinyl)phenyl]-β-methylacrylsäuremethylester [EP280185, Nr. 136]

Zu einer Lösung von 10 g (0,041 mol) 2-Bromphenylglyoxylsäuremethylester und 6,3 g (0,061 mol) Phenylacetylen in 50 ml Triethylamin gibt man 300 mg Palladium(II)acetat, 1,5 g Triphenylphosphin und 100 mg Kupfer(I)jodid. Nachdem man 30 min lang Stickstoff durch die Lösung geleitet hat, erhitzt man 90 min auf 90°C. Anschließend wird eingeengt und der Rückstand in Methylenchlorid aufgenommen. Es wird mit Wasser gewaschen, getrocknet und eingeengt. Der verbleibende Rückstand wird an Kieselgel mit Hexan/ Methyl-tert.-butylether chromatographiert. Nach dem Einengen verbleiben 5,5 g "α-Ketoester" als braunes Öl, ¹H-NMR (CDCl₃/TMS): δ = 2,82 (s, 3H, CH₃); 7,36 - 7,88 ppm (m, 9H, Aryl).

Zu einer Lösung von 8,7 g (0,021 mol) Ethyl-triphenylphosphoniumjodid in 60 ml trockenem Tetrahydrofuran gibt man bei 5 °C 2,4 g (0,021 mol) Kalium-tert.butylat. Man rührt 1 Stunde bei dieser Temperatur nach und tropft dann eine Lösung von 5,5 g (0,021 mol) "α-Ketoester" (s.o.) in 20 ml Tetrahydrofuran bei 5°C zu und rührt 3 Stunden bei Raumtemperatur. Der Reaktionsansatz wird auf Wasser gegossen, mit Methylenchlorid extrahiert, getrocknet und eingeengt. Chromatographie an Kieselgel mit Hexan/Methyl-tert.-butylether liefert 1,2 g der Verbindung [EP280185, Nr. 136] als braunes Öl. E/Z-Gemisch. ¹H-NMR (CDCl₃/TMS): δ = 1,74 (d, 3H, CH₃-Isomer A); 2,22 (d, 3H, CH₃-Isomer B); 3,70; 3,71 (s, 3H, CH₃-Isomer A, B); 6,38 (q, 1H, CH); 7,17 - 7,61 ppm (m, 9H, Aryl).

### Beispiel 4

### Darstellung von 2 Ethinylphenylglyoxylsäure-N-methylamid-O-methyloxim

Zu 20 ml einer 40 %igen Methylaminlösung gibt man 1 g (4,6 mmol) des terminalen Acetylens (dargestellt in Beispiel 1) und rührt 45 min bei 40 - 50°C. Die Lösung wird mit Methyl-tert-butylether extrahiert. Man trocknet und engt ein. Es verbleiben 700 mg (70 %) der Verbindung als farbloser Feststoff.

¹H-NMR (CDCl₃/TMS): δ = 2,99 (d, 2H, NCH₃); 3,13 (s, 1H, ≡C-H), 3,98 (s, 3H, OCH3), 6,80 (br, 1H, NH); 7,21 - 7,58 ppm (m, 4H).

### Beispiel 5

### Darstellung von 2-Benzoyl-ethinylphenylglyoxylsäuremethylester-O-methyloxim (Verbindung 26, Tabelle 6)

Zu einer Mischung bestehend aus 50 mg Kupfer(II)chlorid, 110 mg Palladium(II)acetat, 380 mg Triphenylphosphin und 2,6 g (18,4 mmol) Benzoylchlorid in 100 ml Triethylamin gibt man unter Stickstoff bei 90°C 2 g (9,2 mmol) Acetylenderivat (dargestellt in Beispiel 1). Nach 30 min läßt man abkühlen und filtriert den Feststoff ab. Das Filtrat wird eingeengt, in Methyl-tert.-butylether aufgenommen, mit Wasser gewaschen, getrocknet und eingeengt. Der Rückstand wird mit Diethylether verrührt und der ausgefallene hellbraune Feststoff abgesaugt. Es verbleiben so 2,0 g (68 %) der Verbindung 26, Tabelle 6.

Schmpkt. 89 - 94°C.

### Beispiel 6

### Darstellung von 2-(Methoxyiminobenzoyl)ethinylphenylglyoxylsäuremethylester-O-methyloxim (Verbindung 10, Tabelle 9).

Eine Mischung aus 1,5 g (4,7 mmol) der Verbindung 26, Tabelle 6 (aus Beispiel 6) und 2,6 g O-Methylhydroxylaminhydrochlorid in 5ml Methanol wird 90 min auf 60°C erhitzt. Man engt ein, nimmt den Rückstand in Methyl-tert.-butylether auf und wäscht mit Wasser. Anschließend wird getrocknet und eingeengt. Der verbleibende Rückstand wird an Kieselgel mit Methyl-tert.-butylether/Hexan-Gemischen chromatographiert. Es verbleiben 1,1 g (67 %) der Verbindung 10, Tab. 9 als gelbes Öl (Isomerengemisch).

¹H-NMR (CDCl₃/TMS): δ = 3,81; 4,04; 4,12 (3H, OCH₃); 7,28 -7,88ppm (9H).

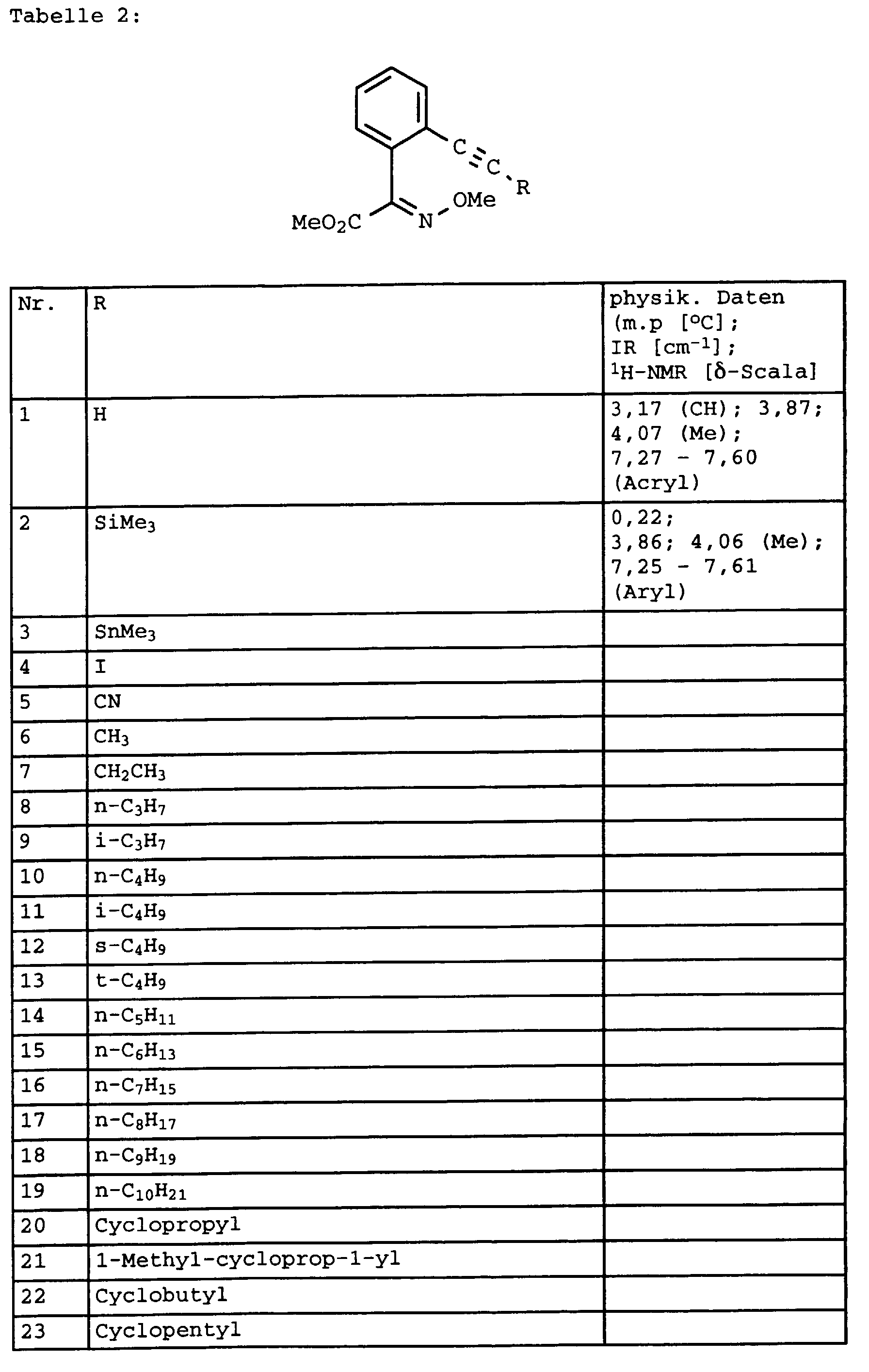

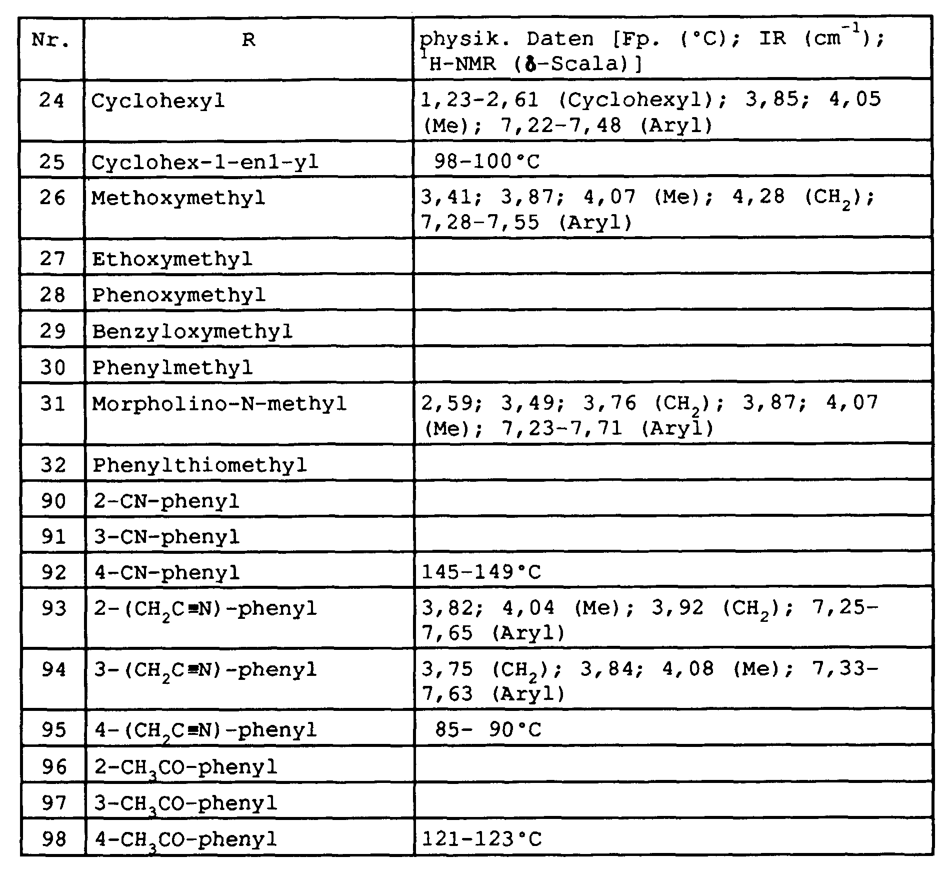

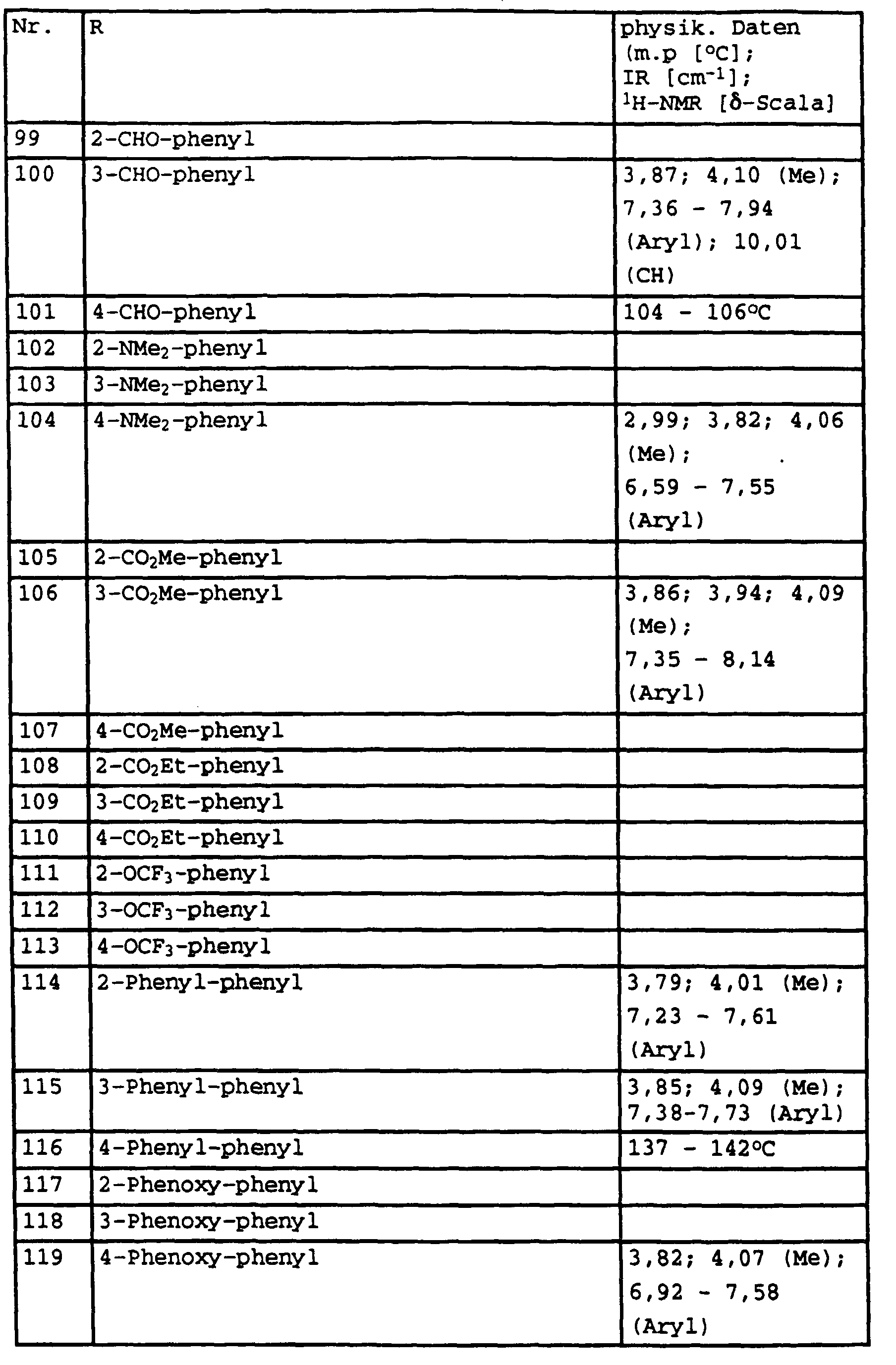

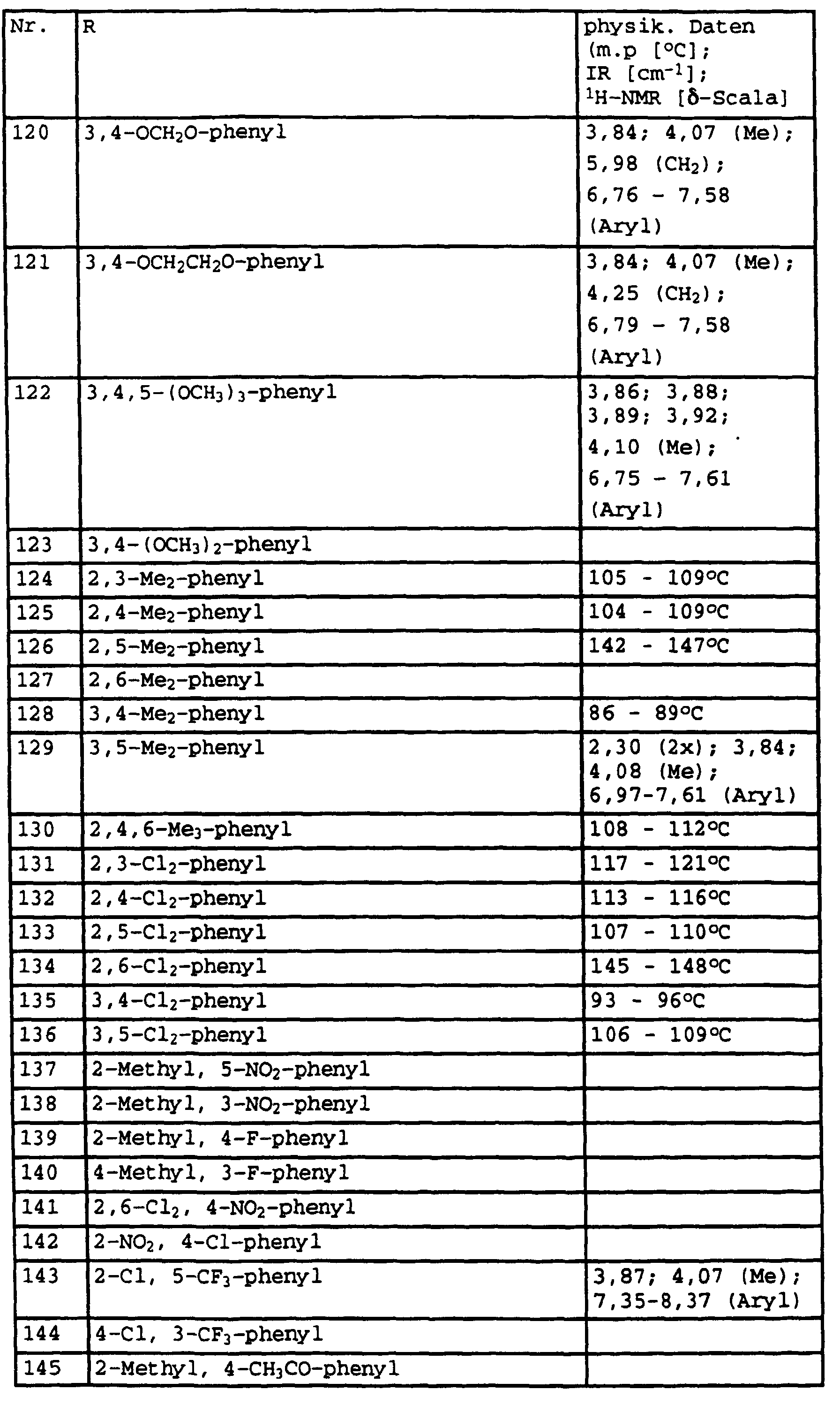

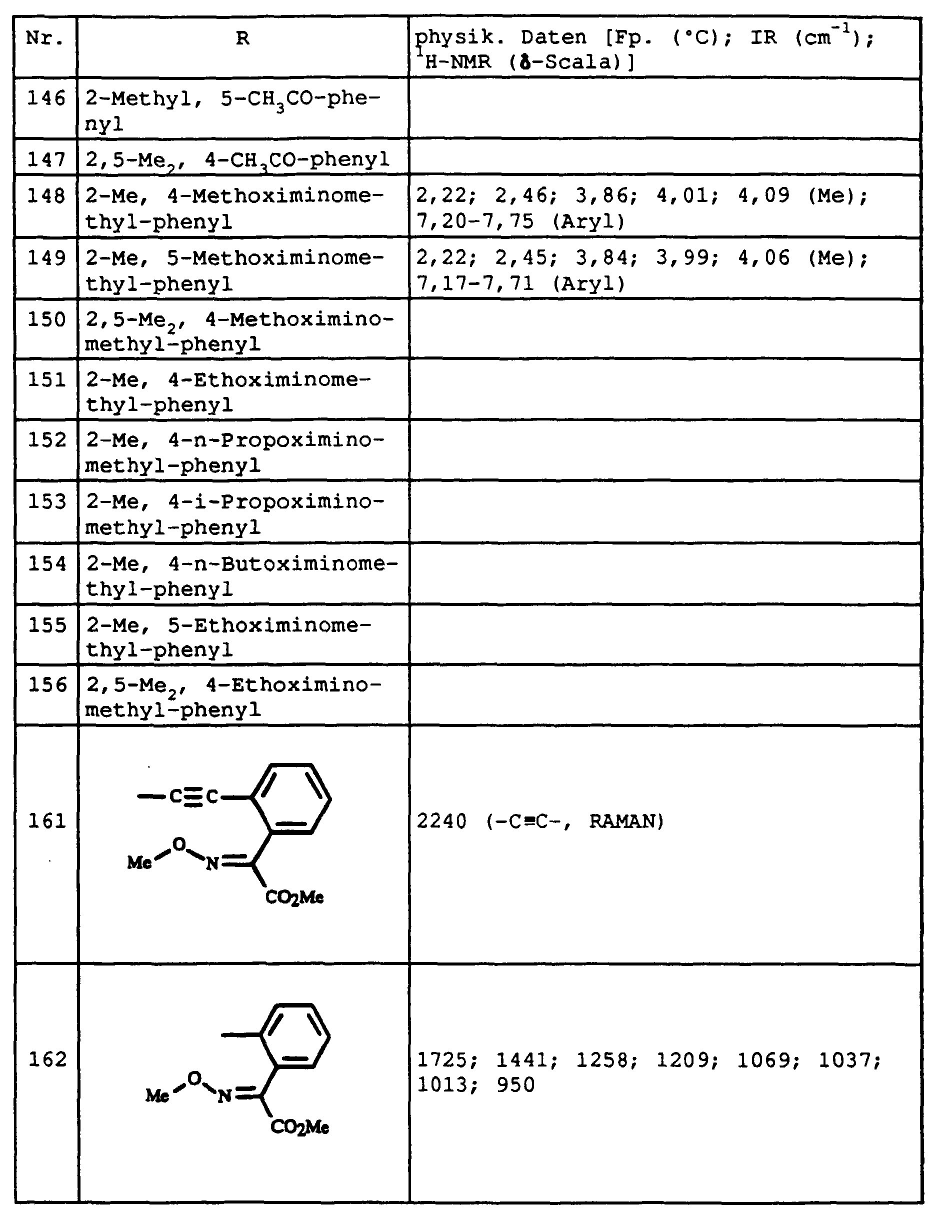

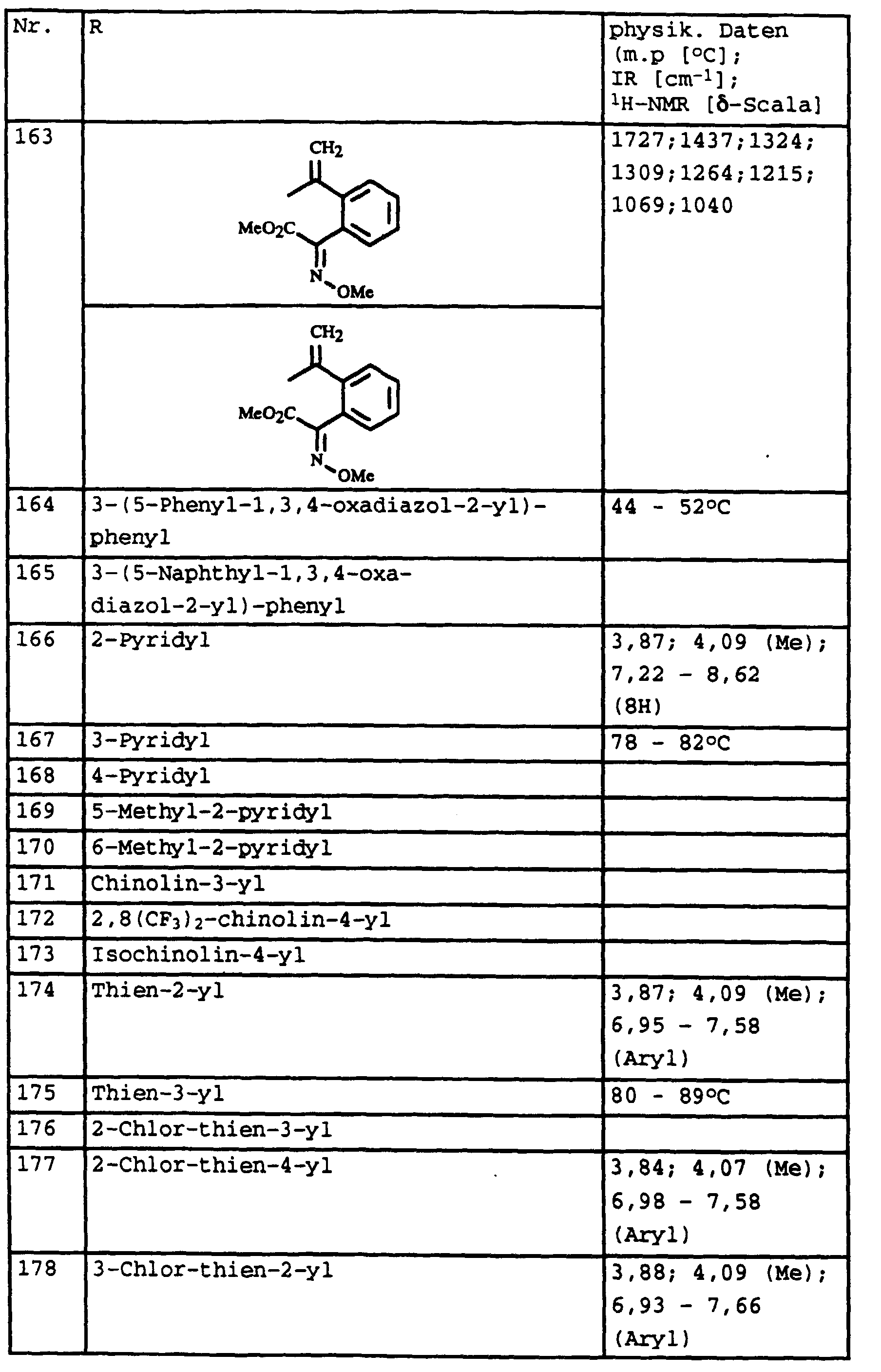

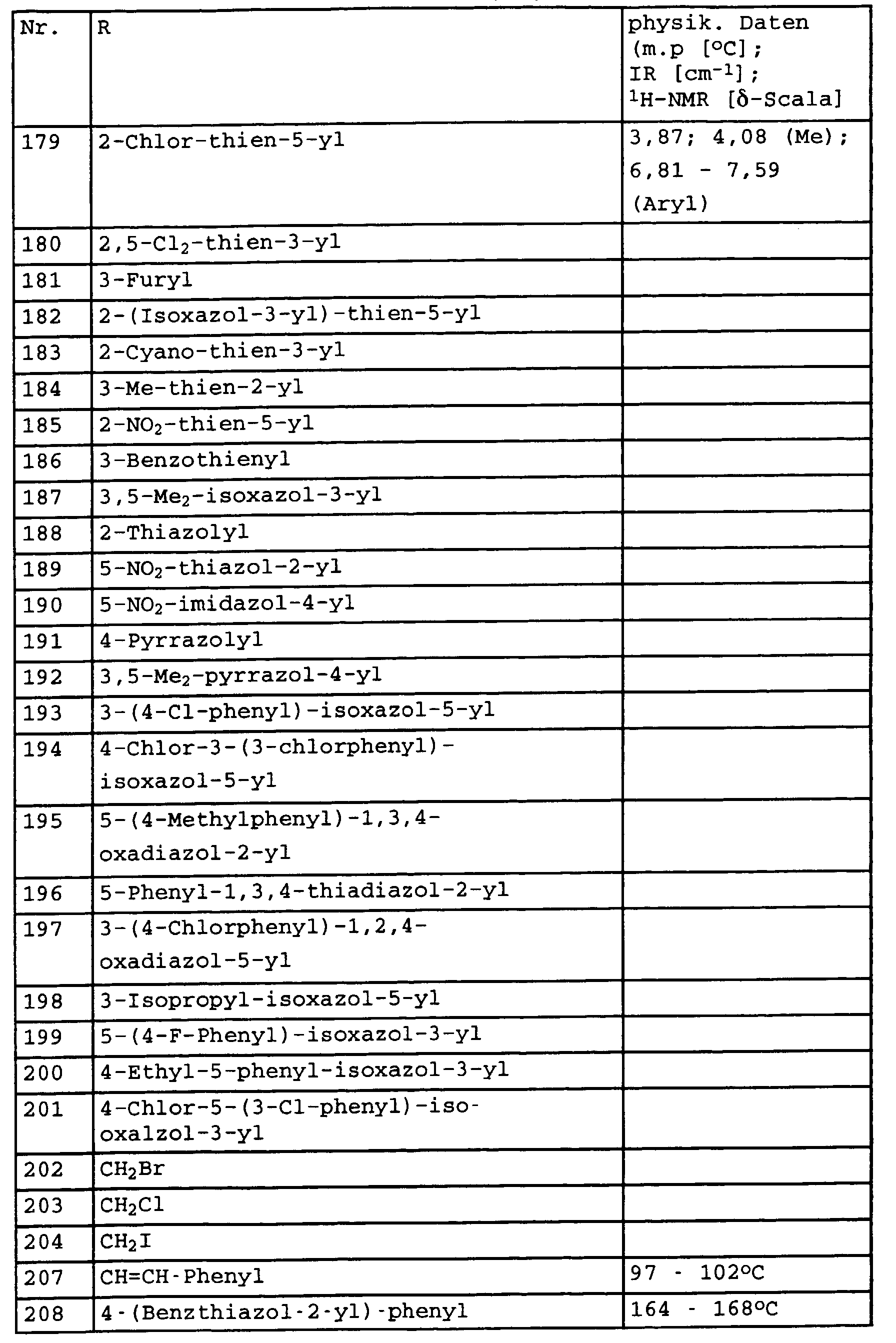

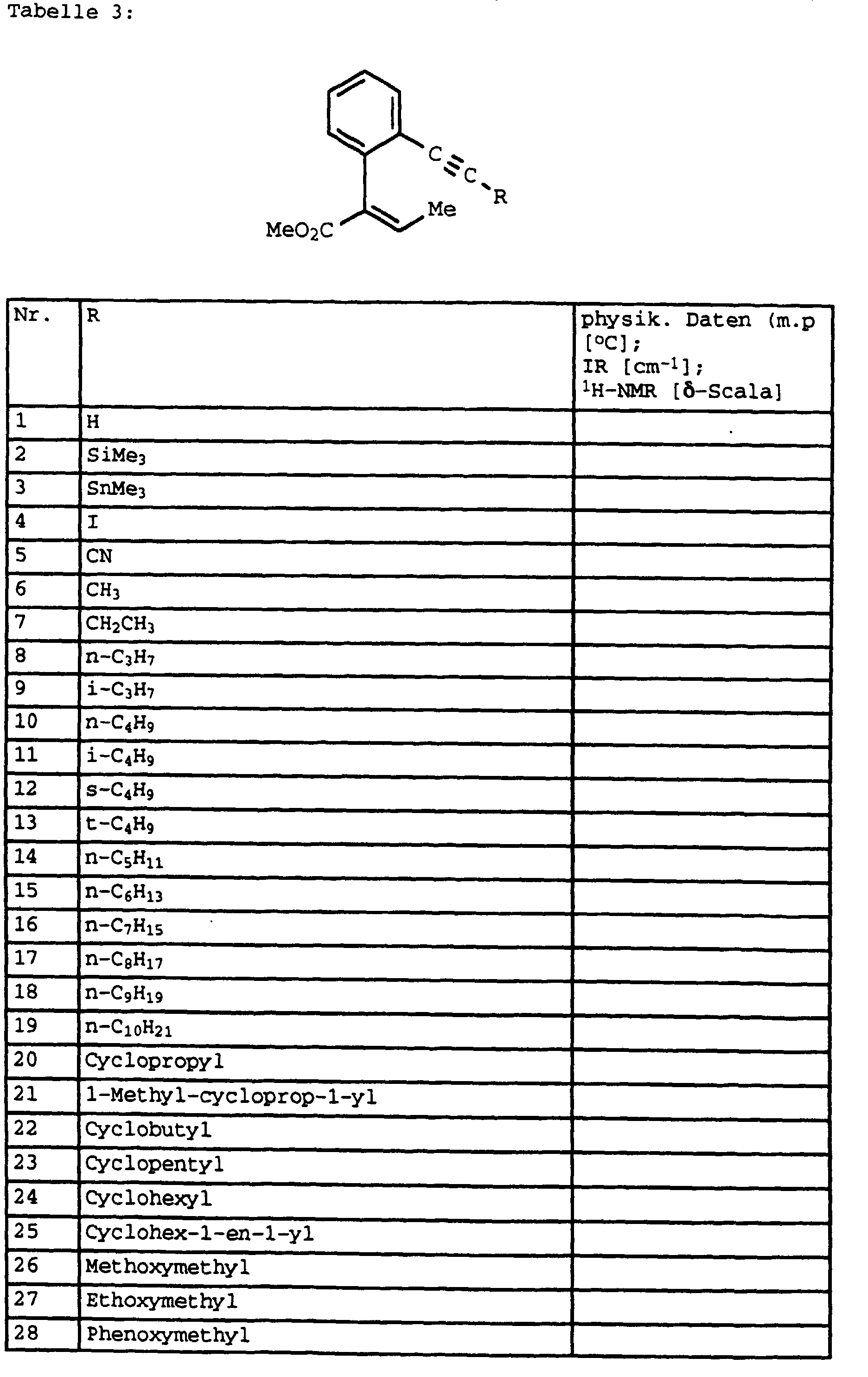

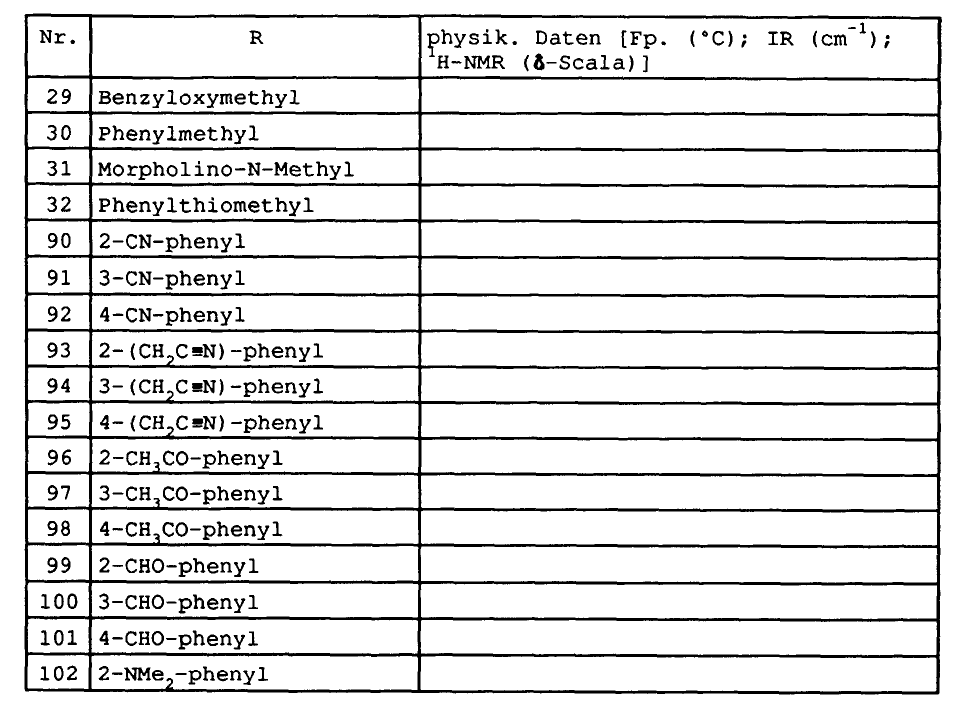

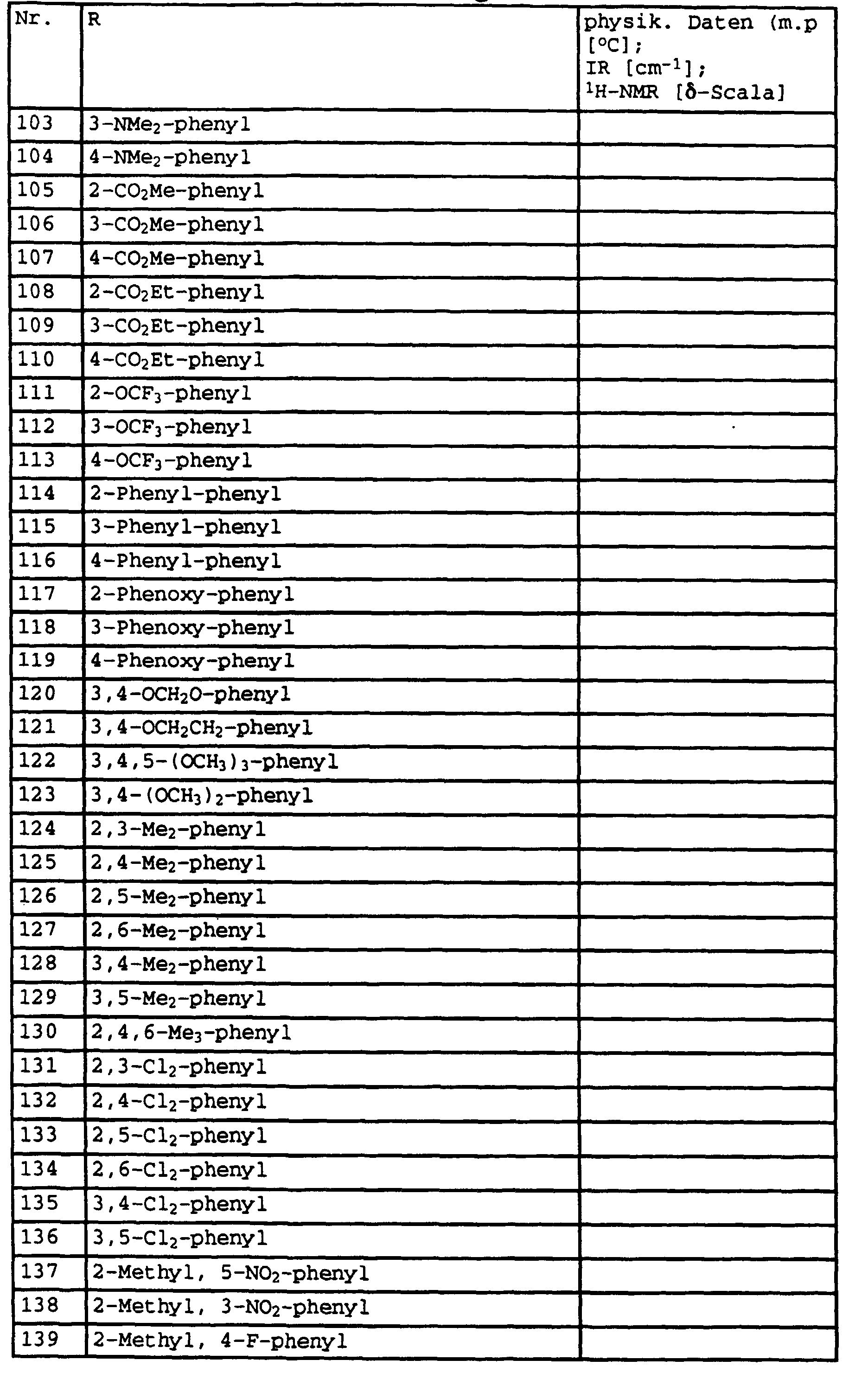

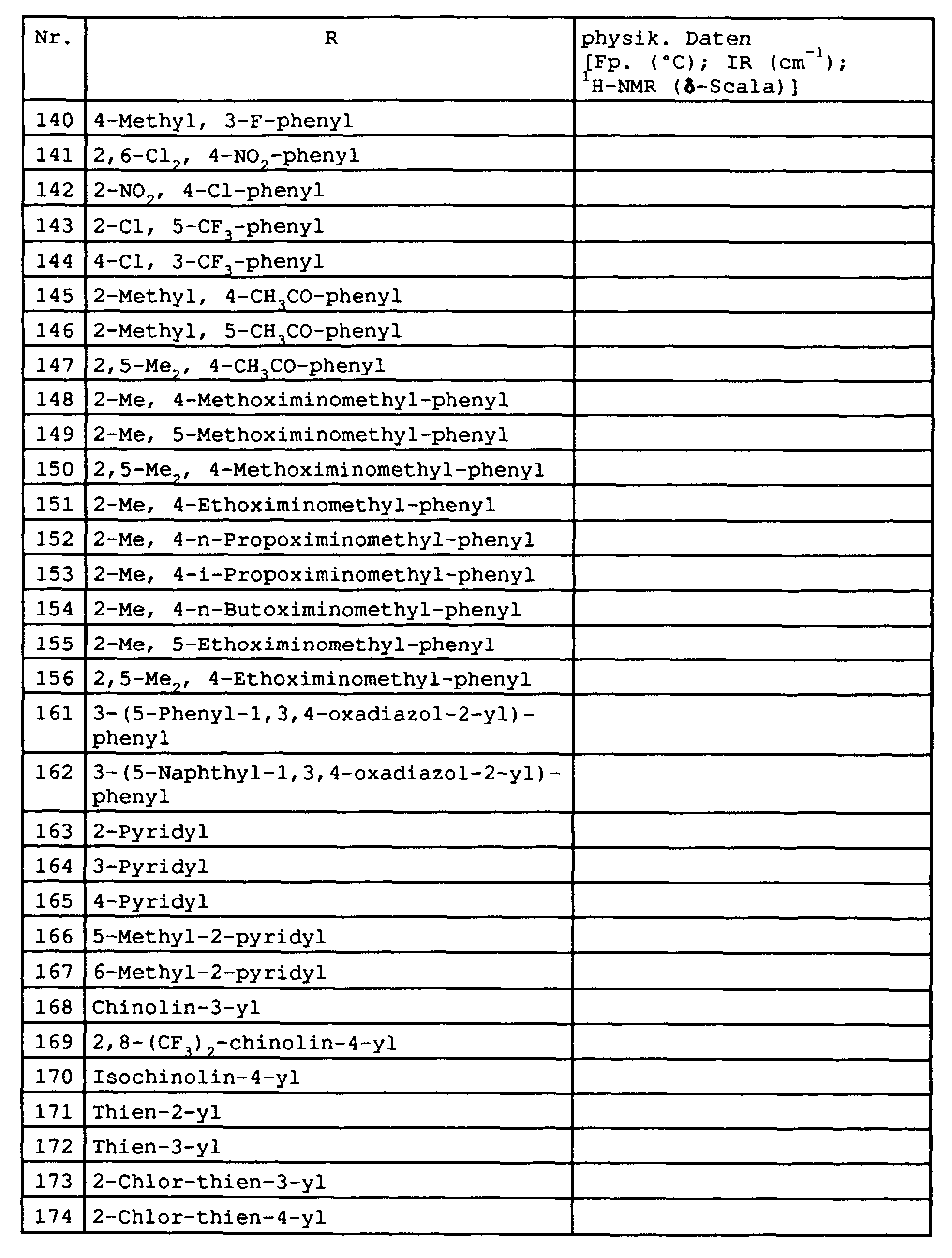

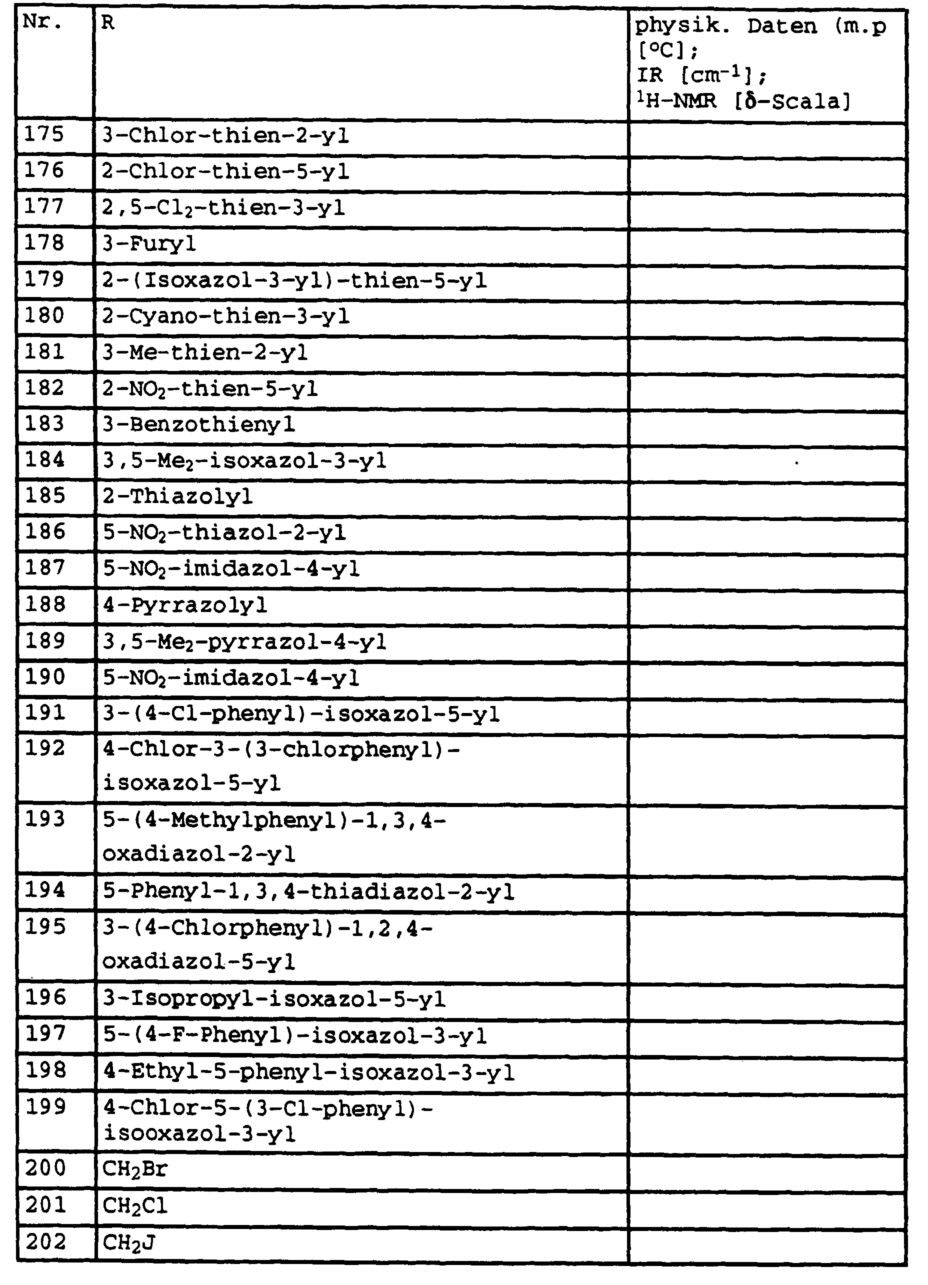

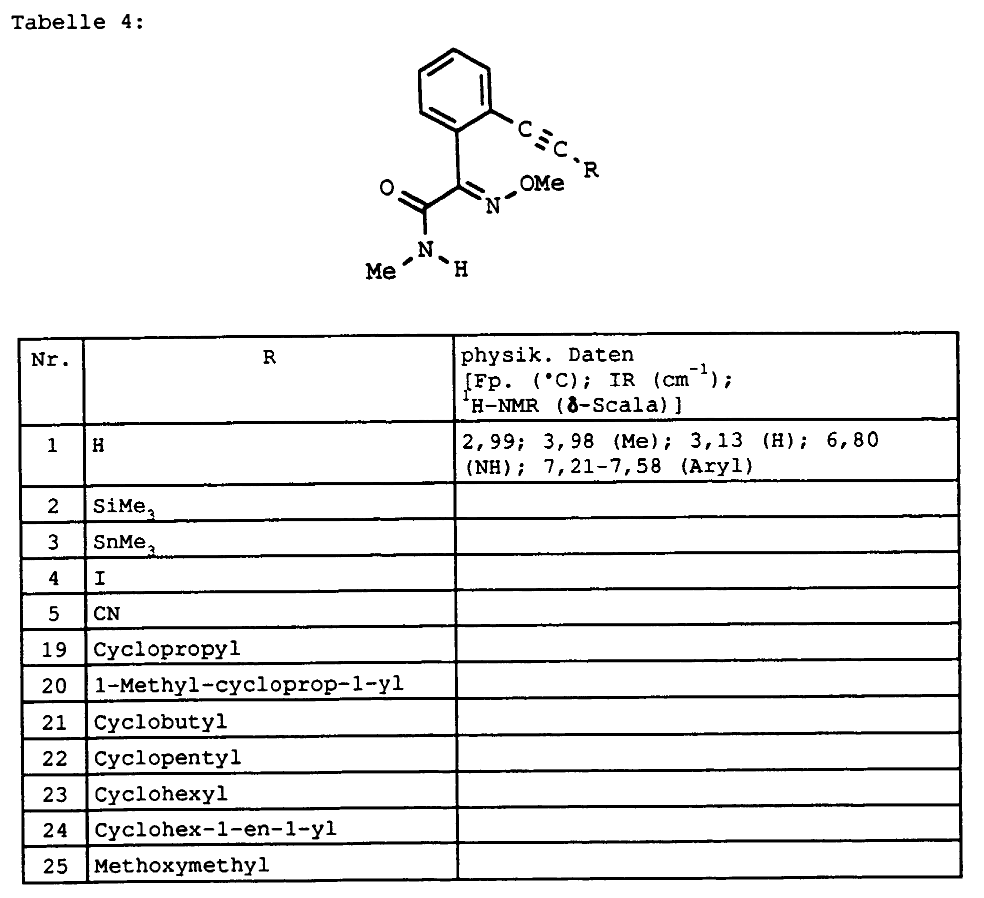

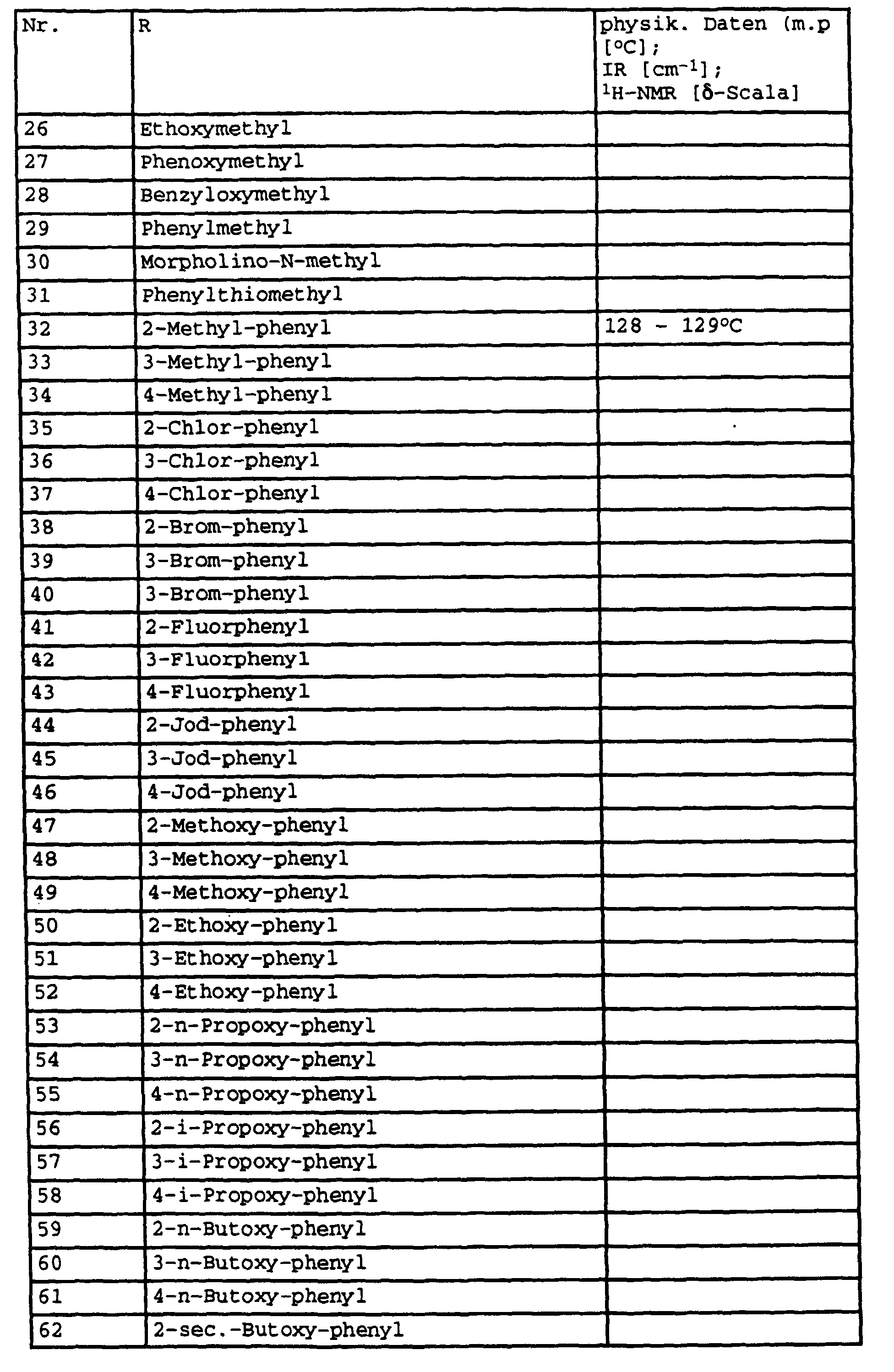

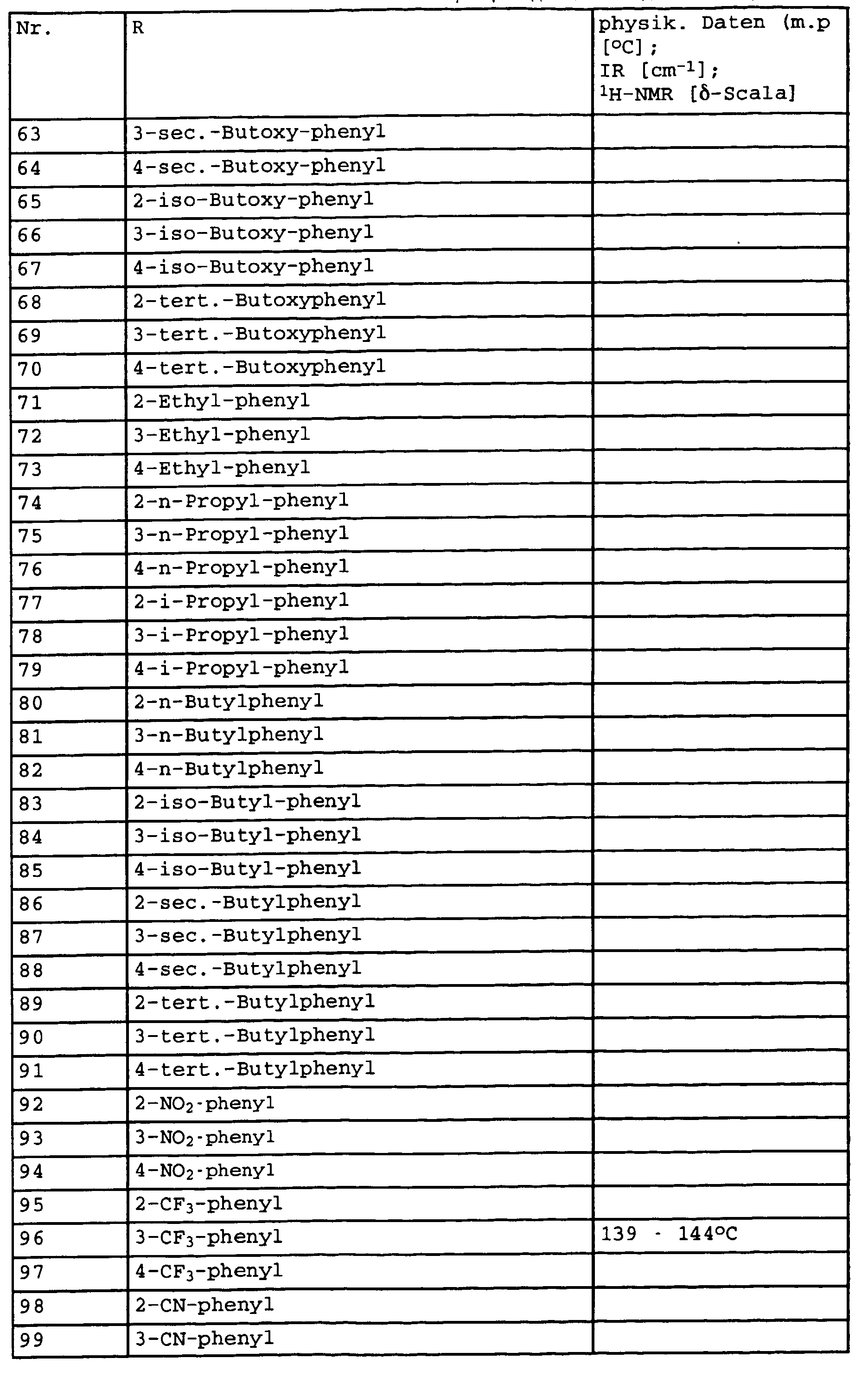

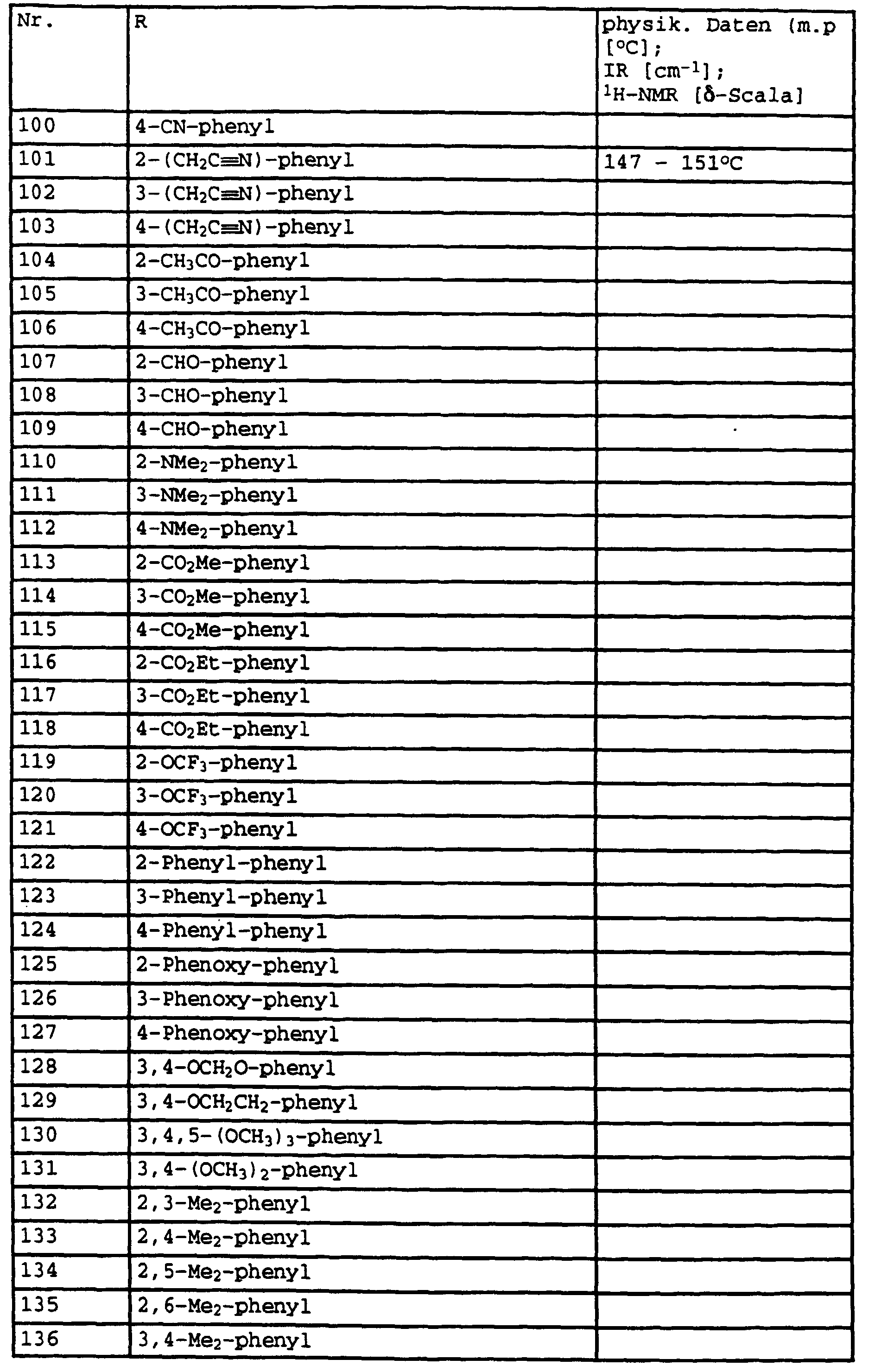

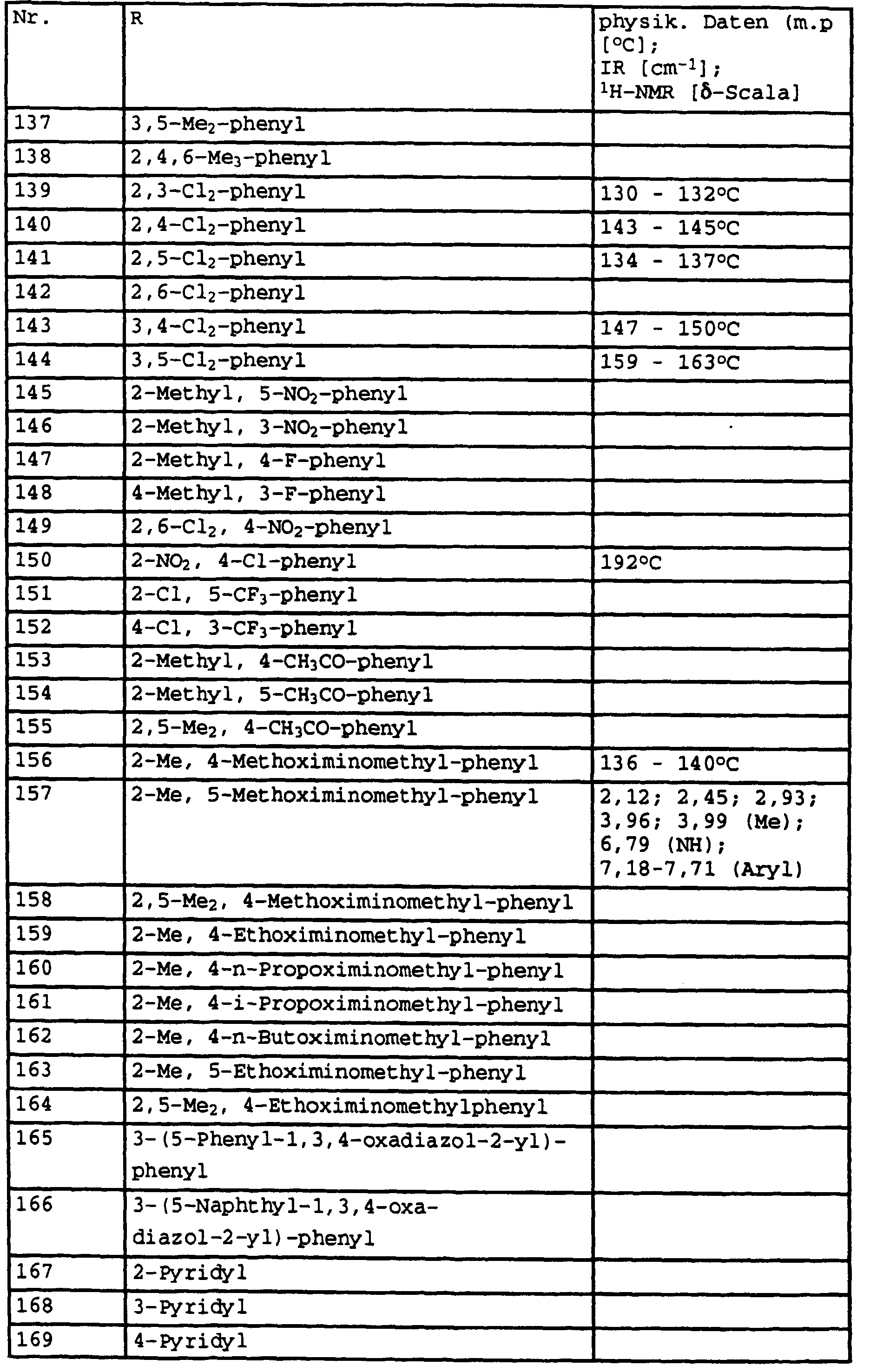

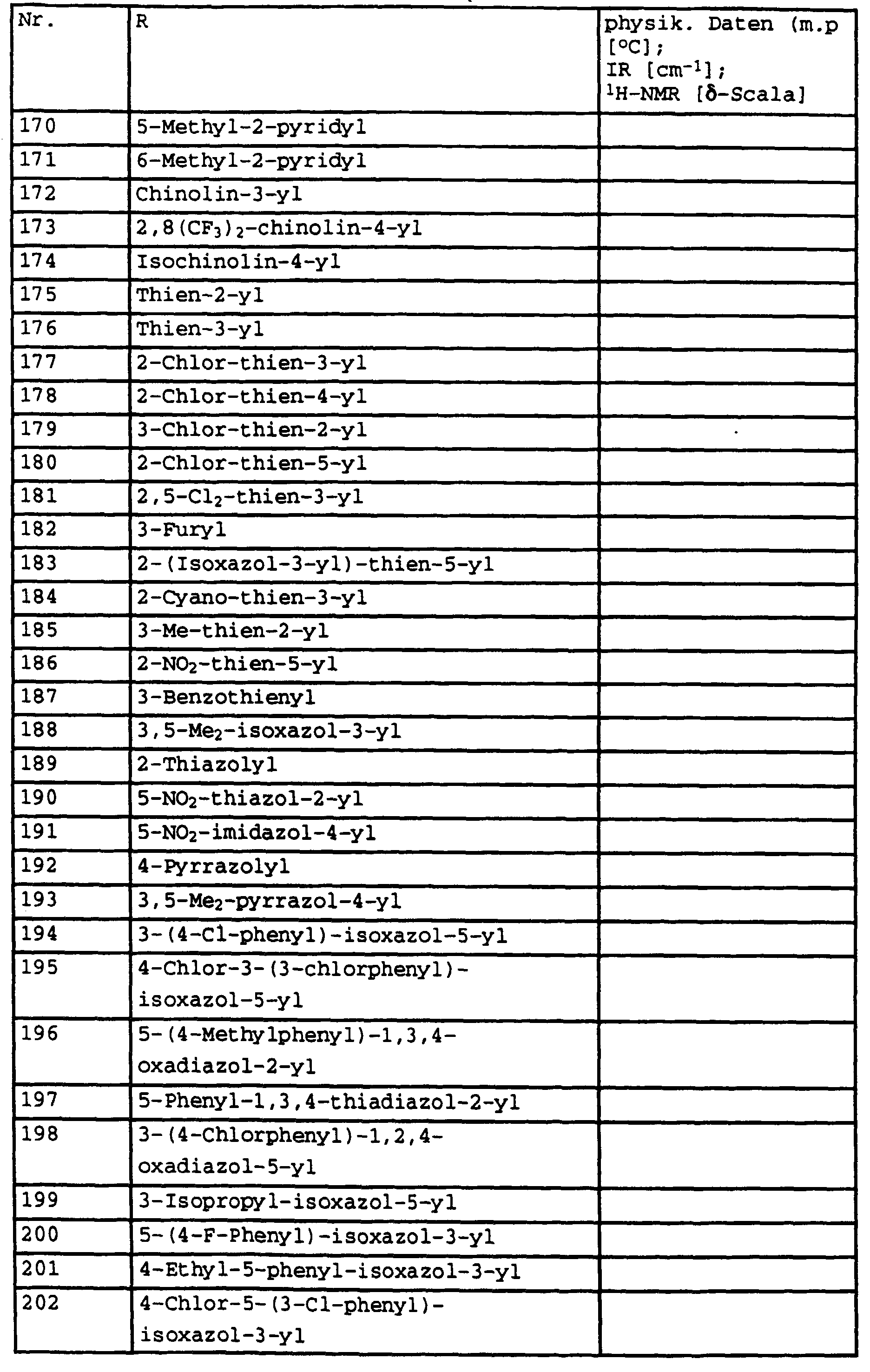

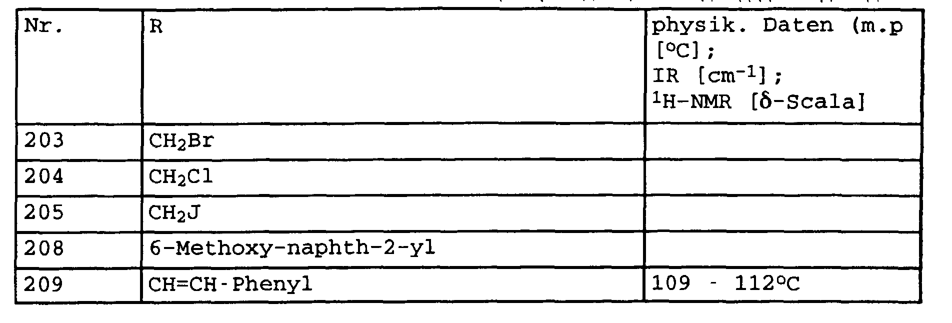

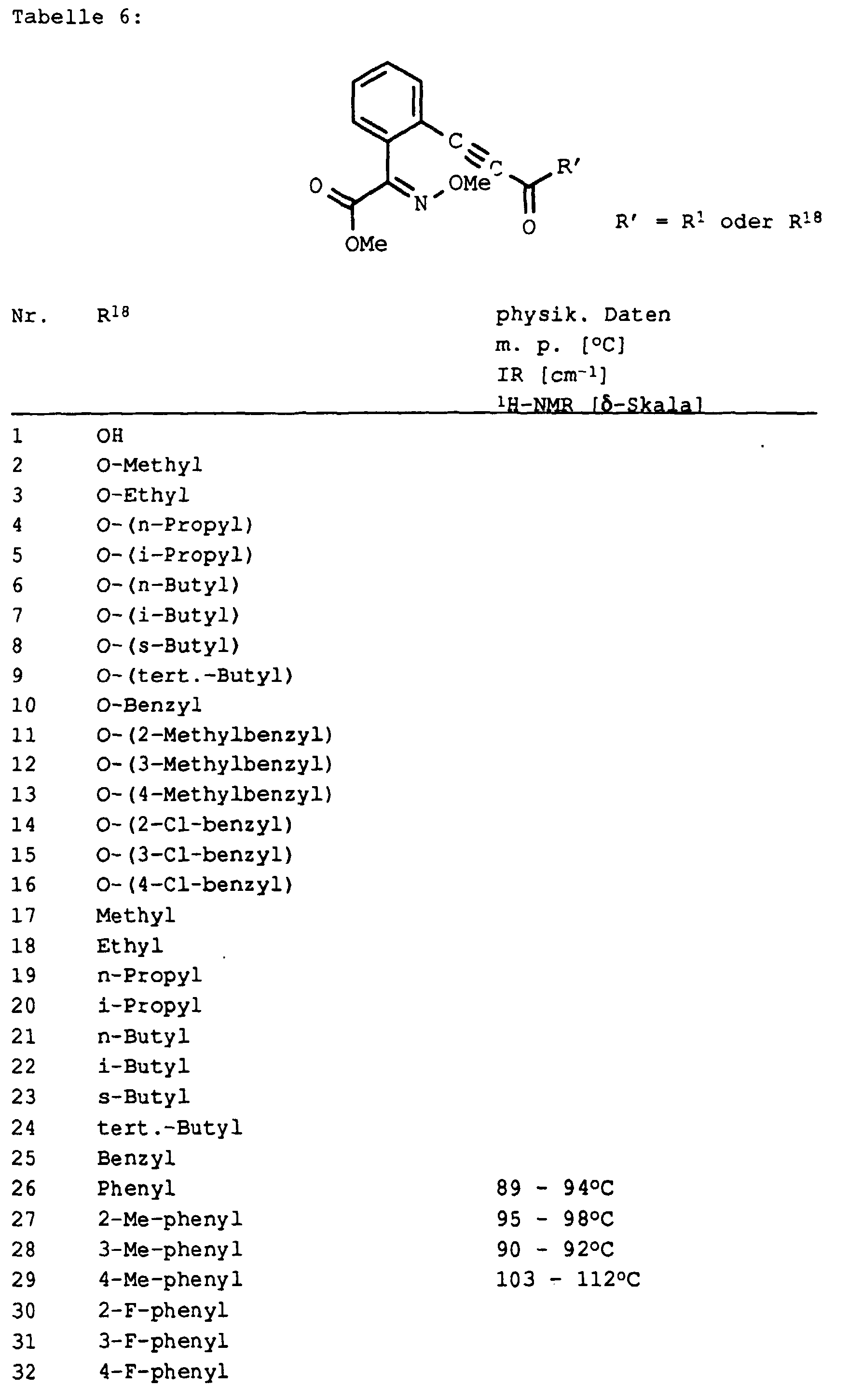

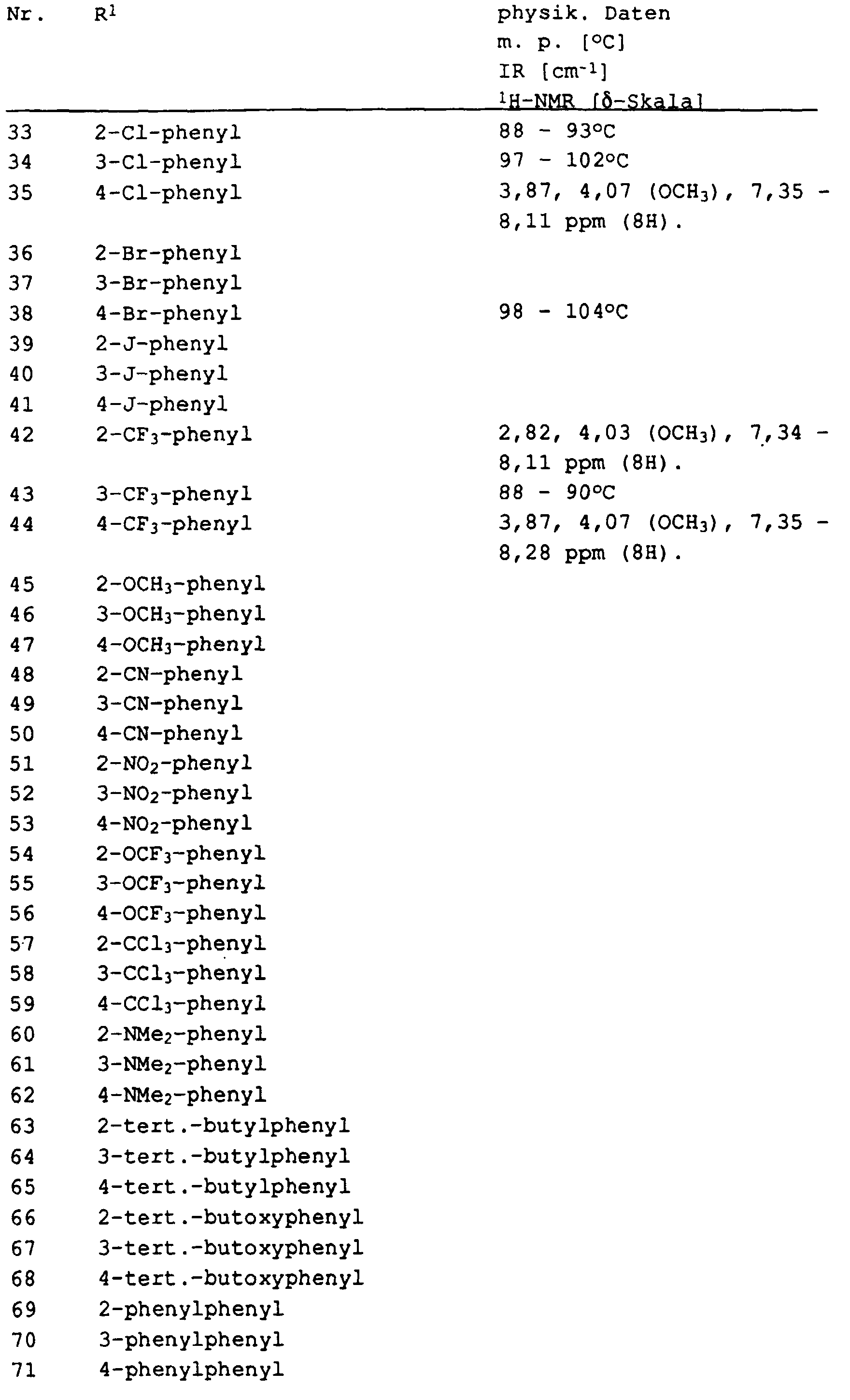

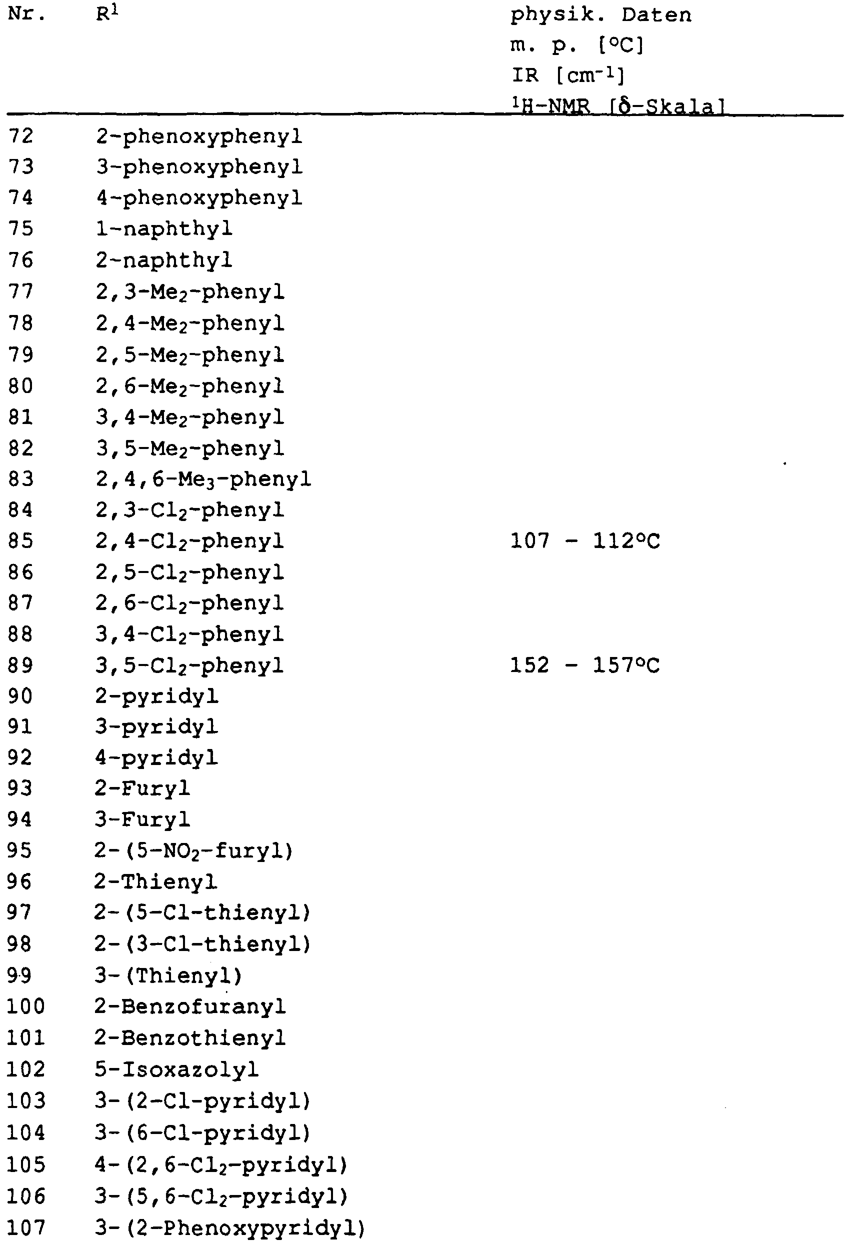

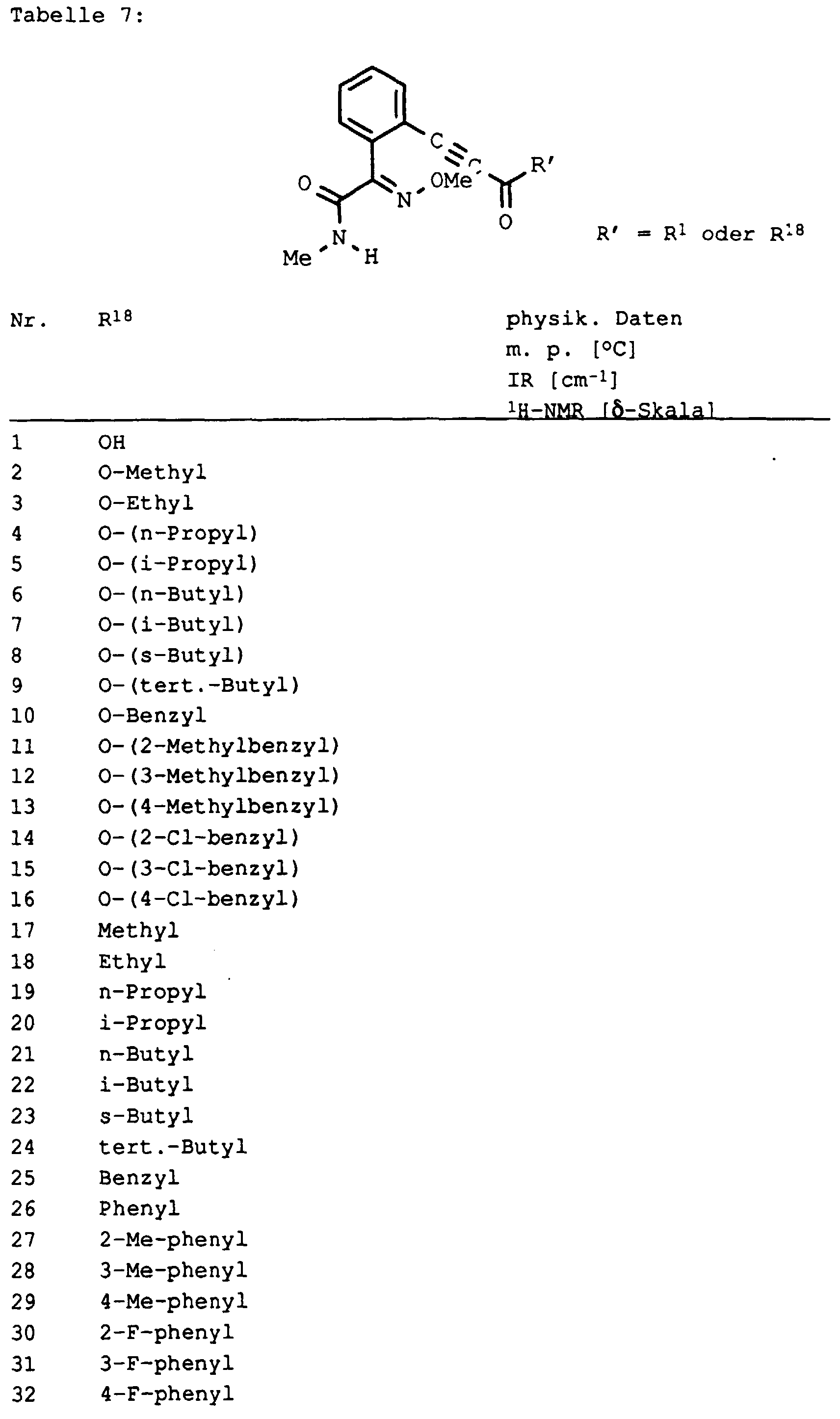

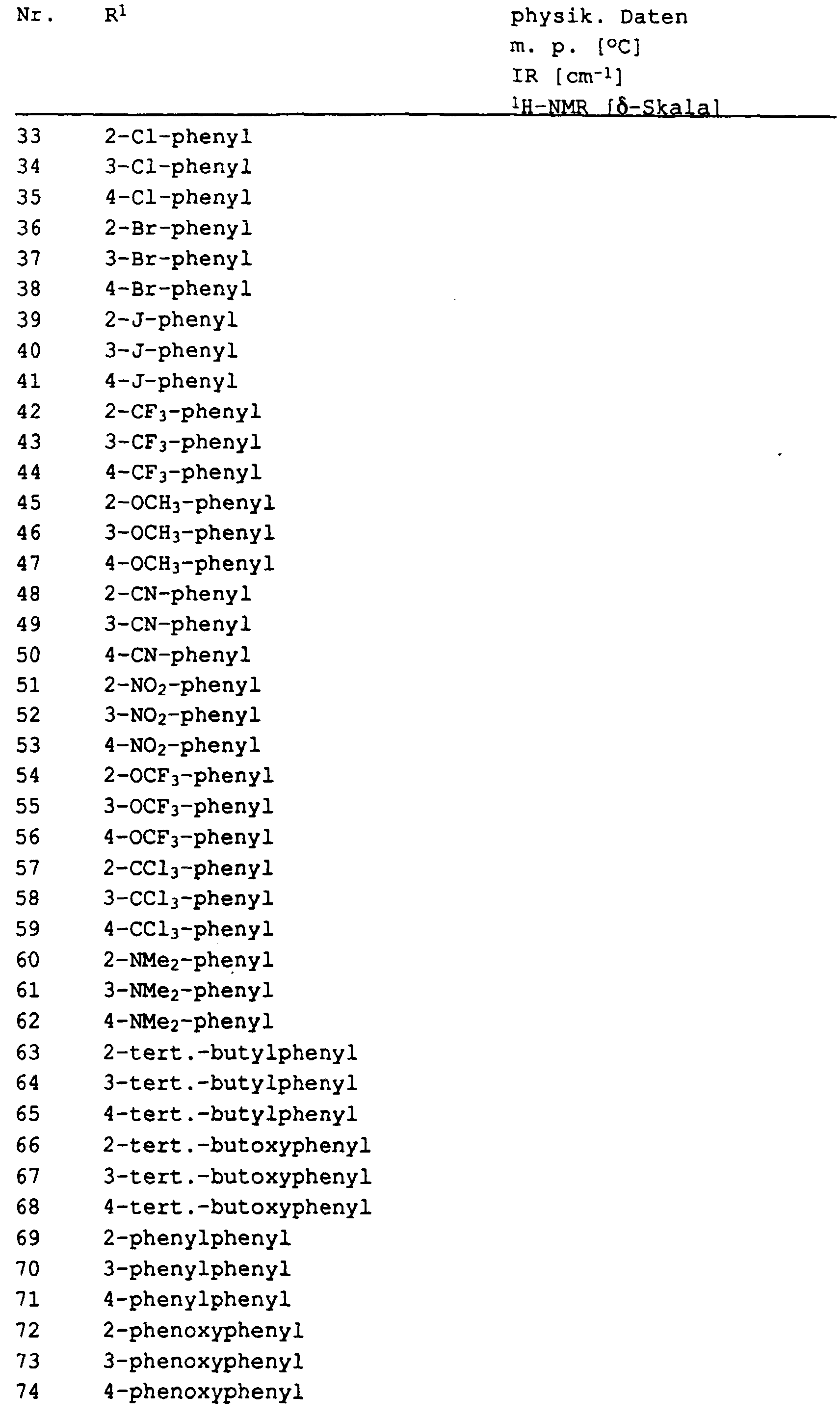

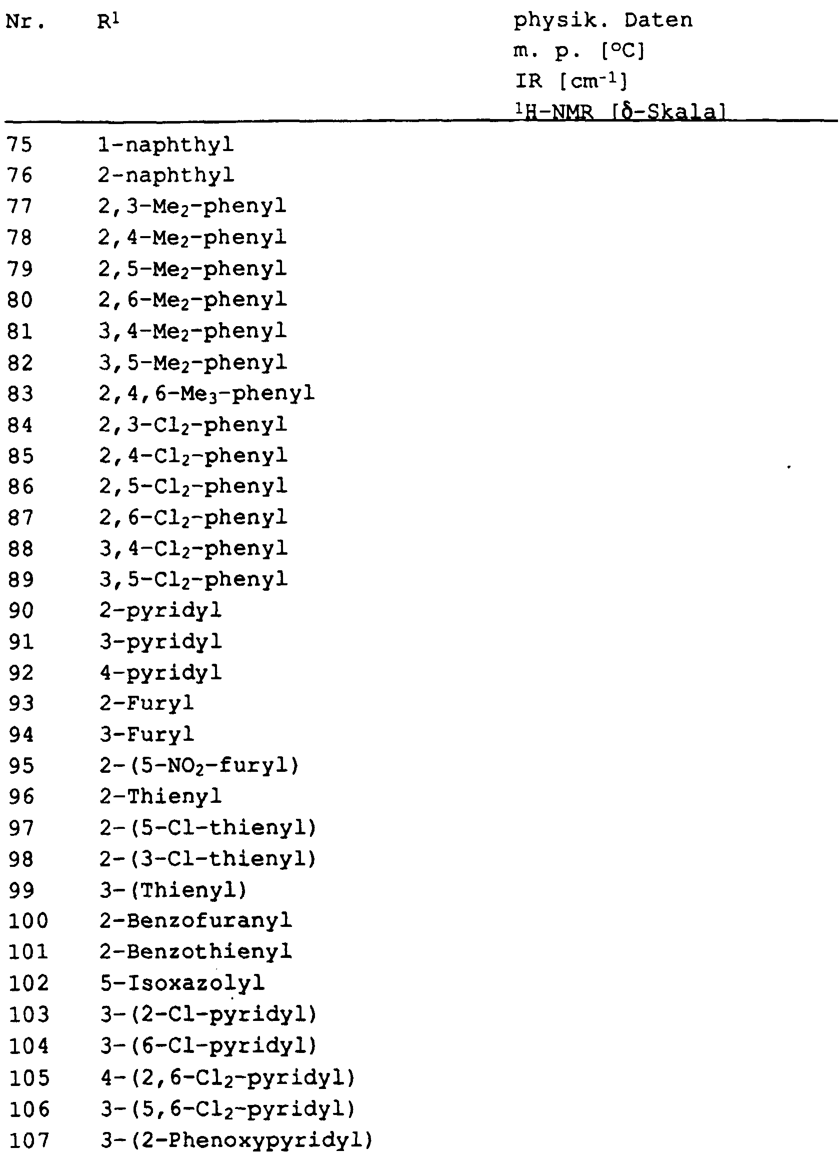

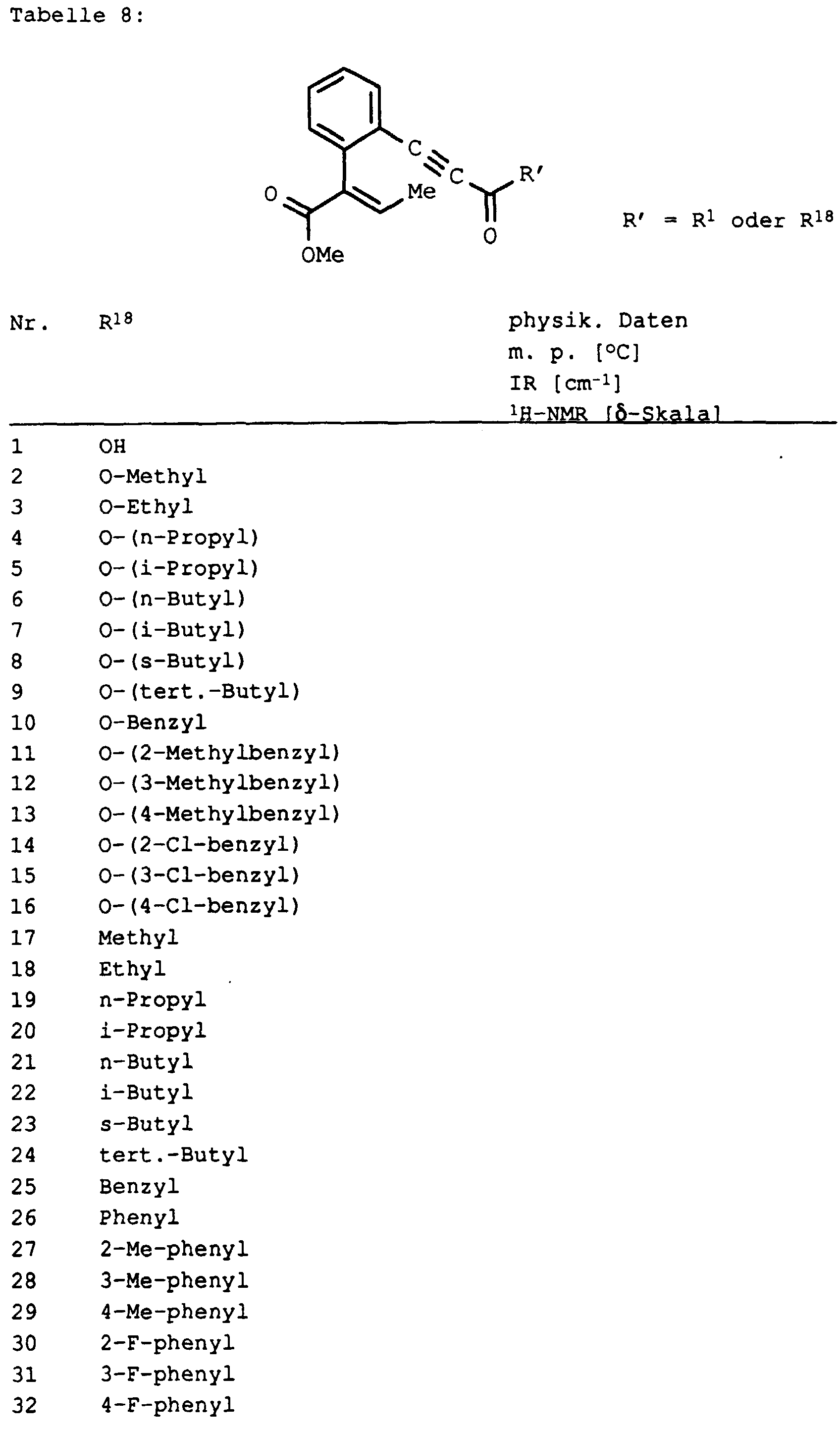

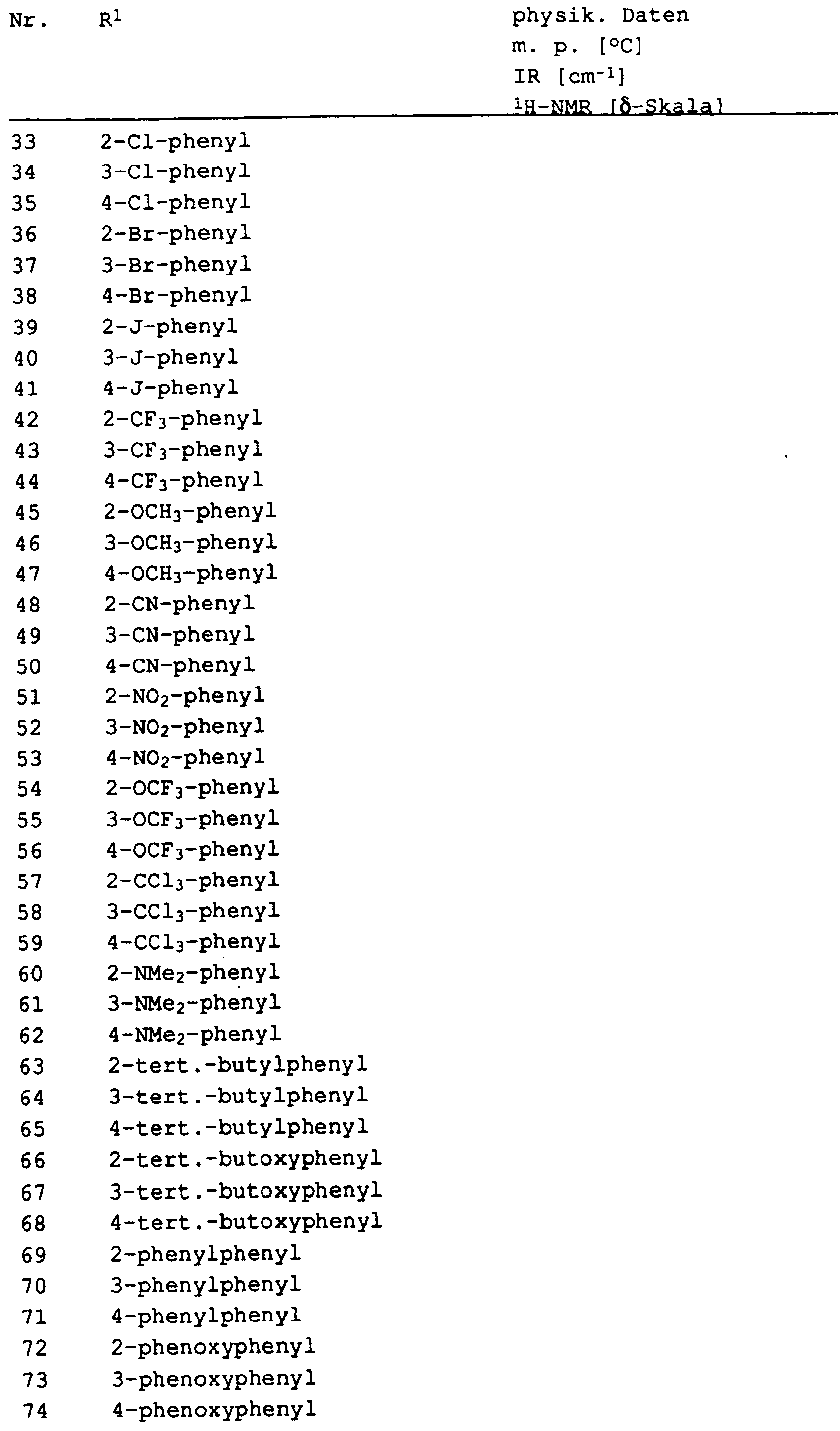

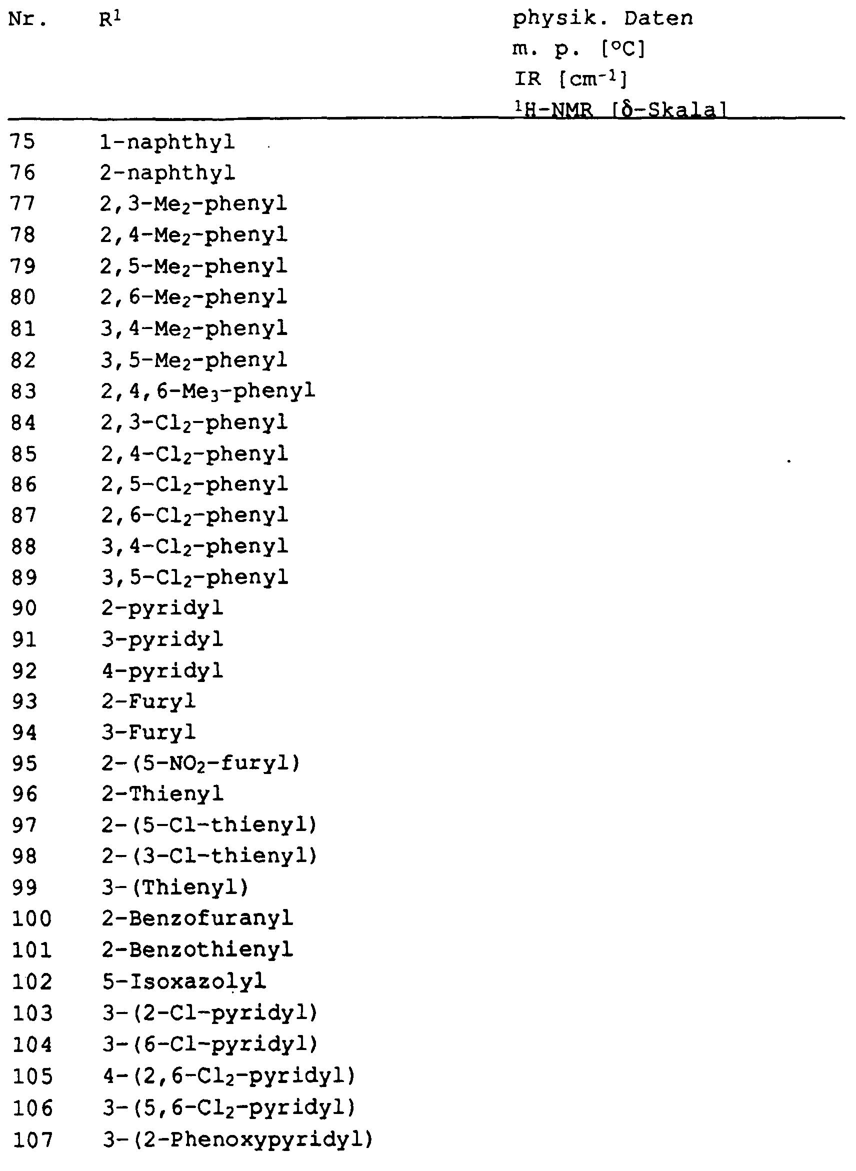

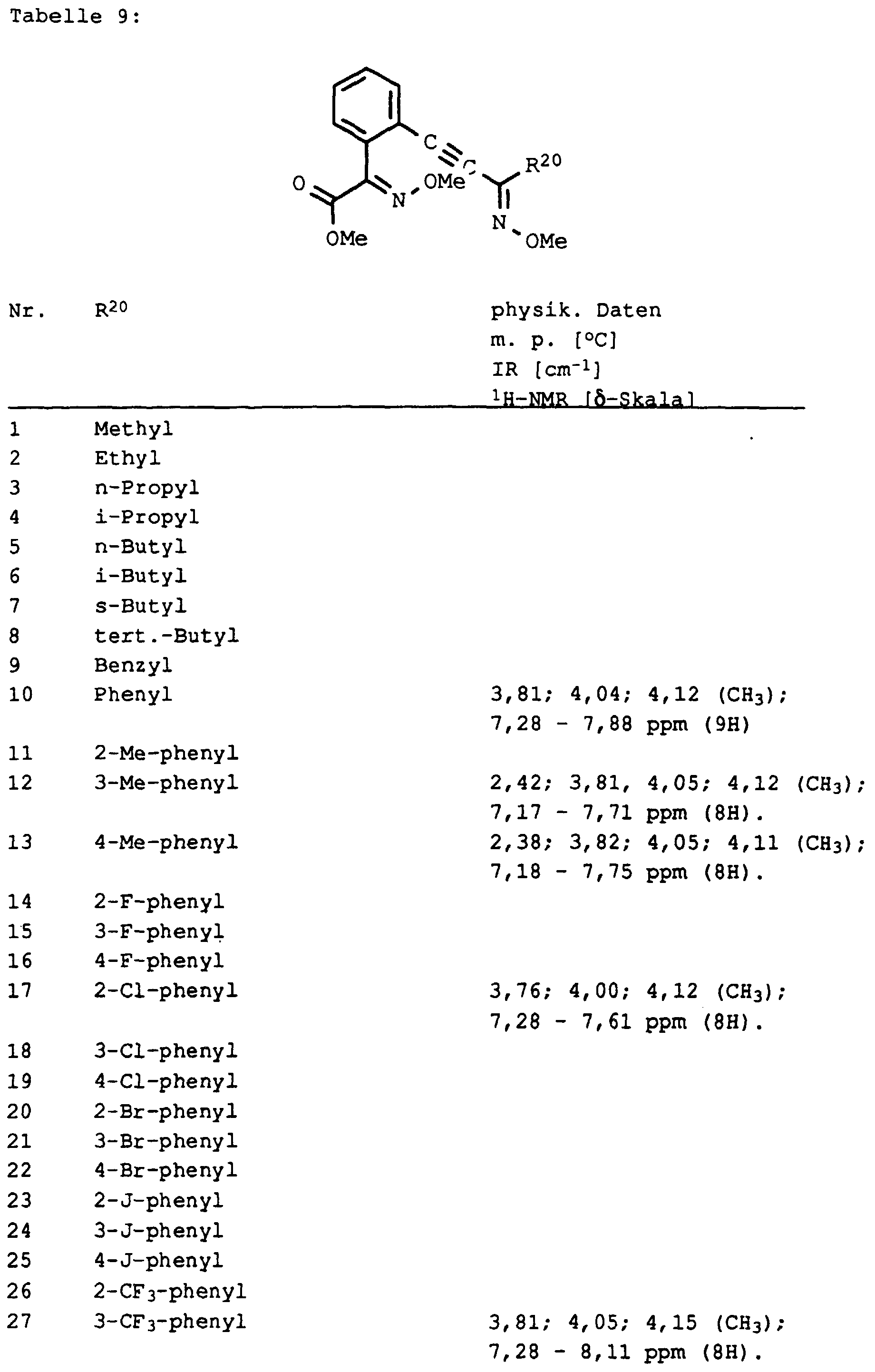

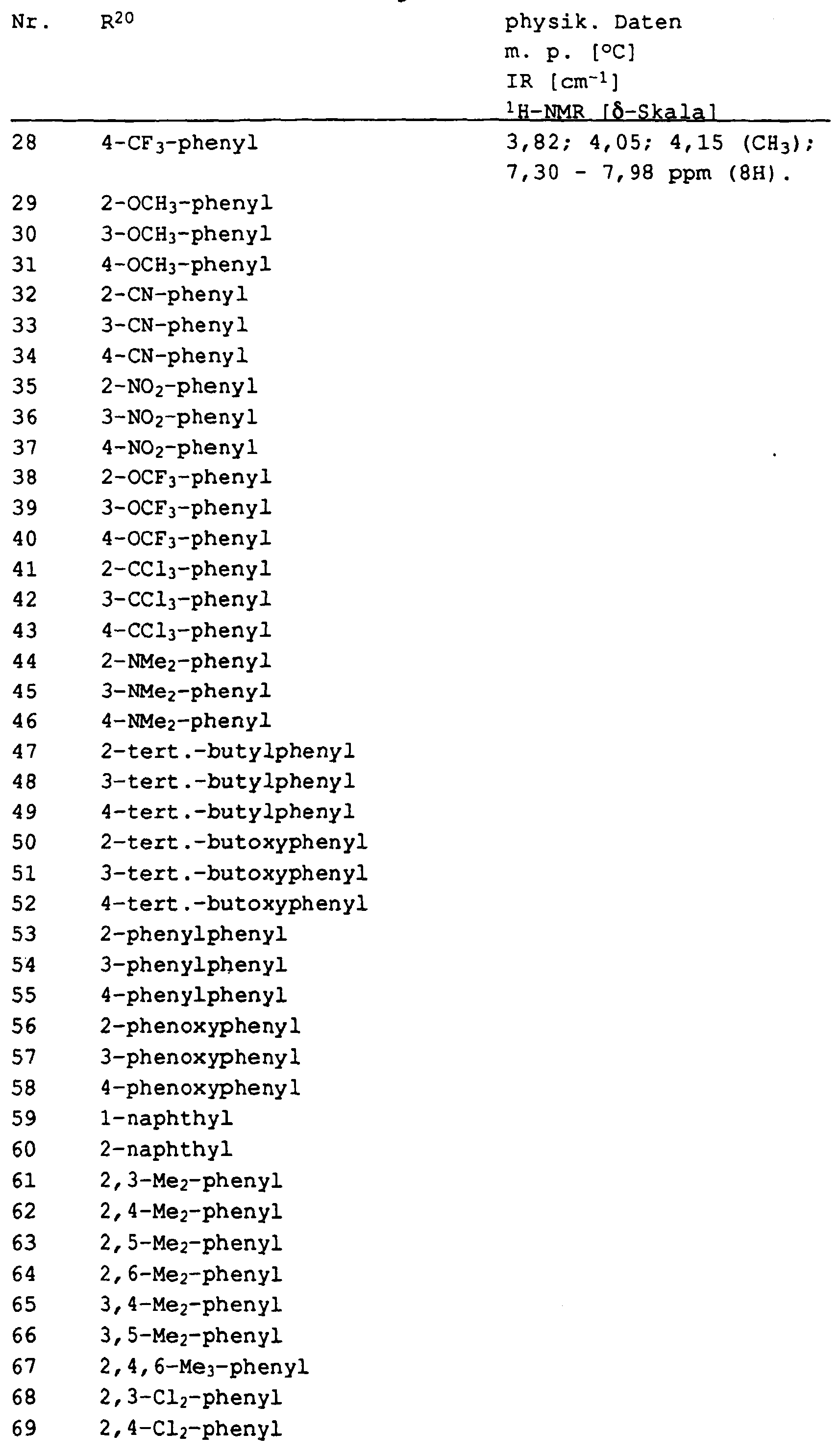

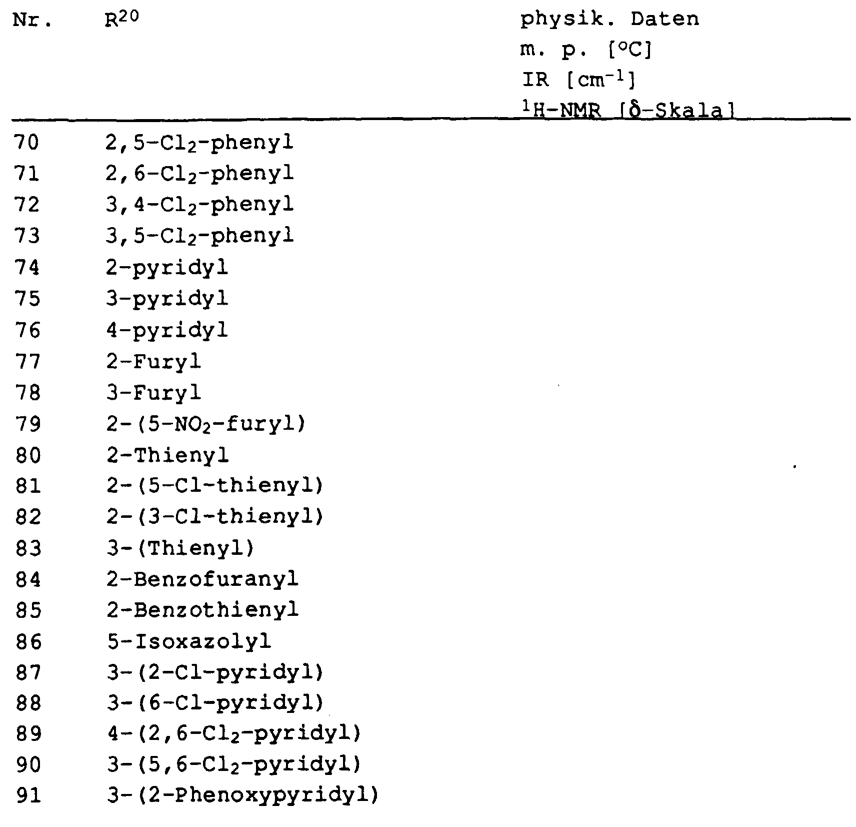

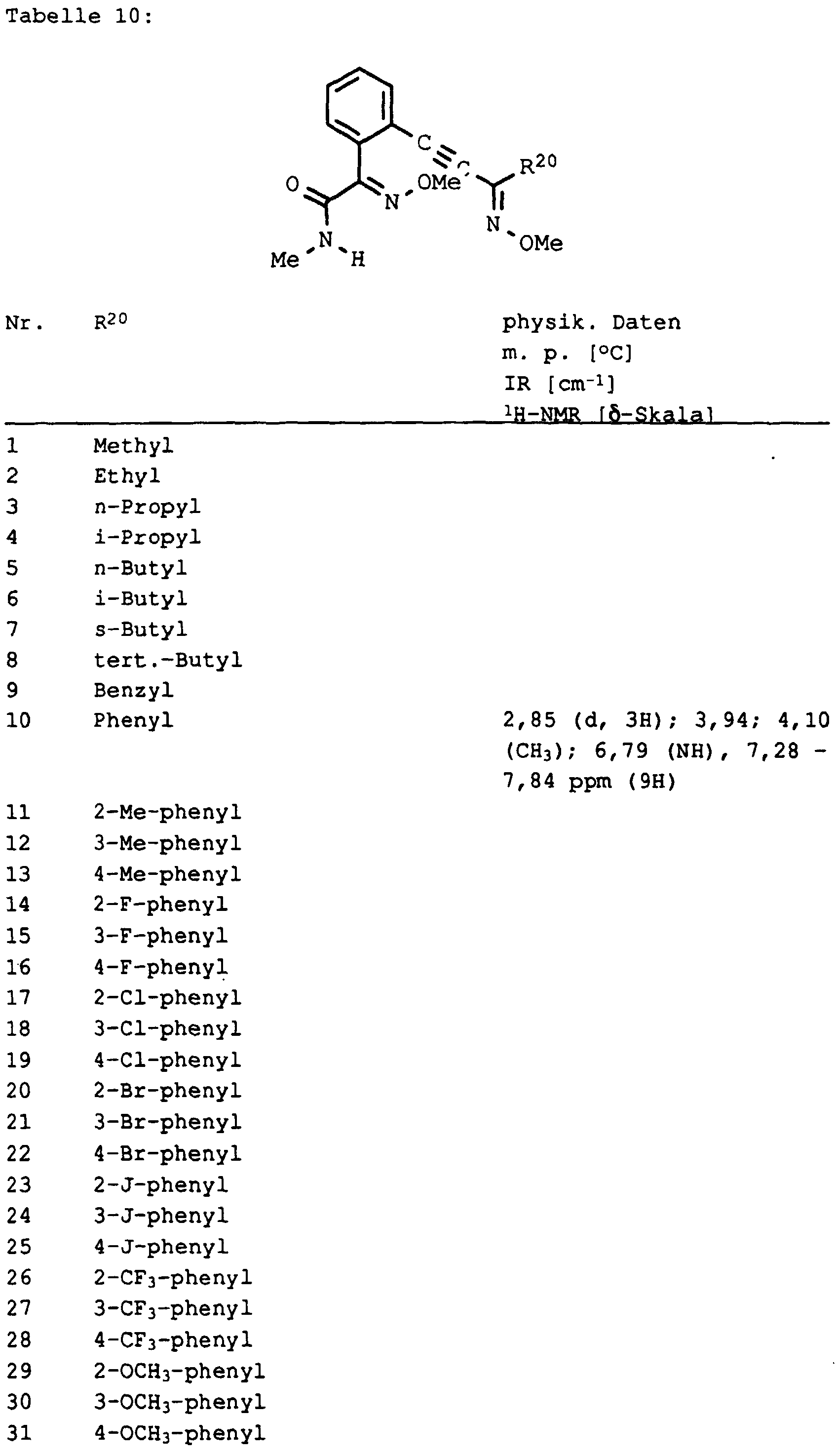

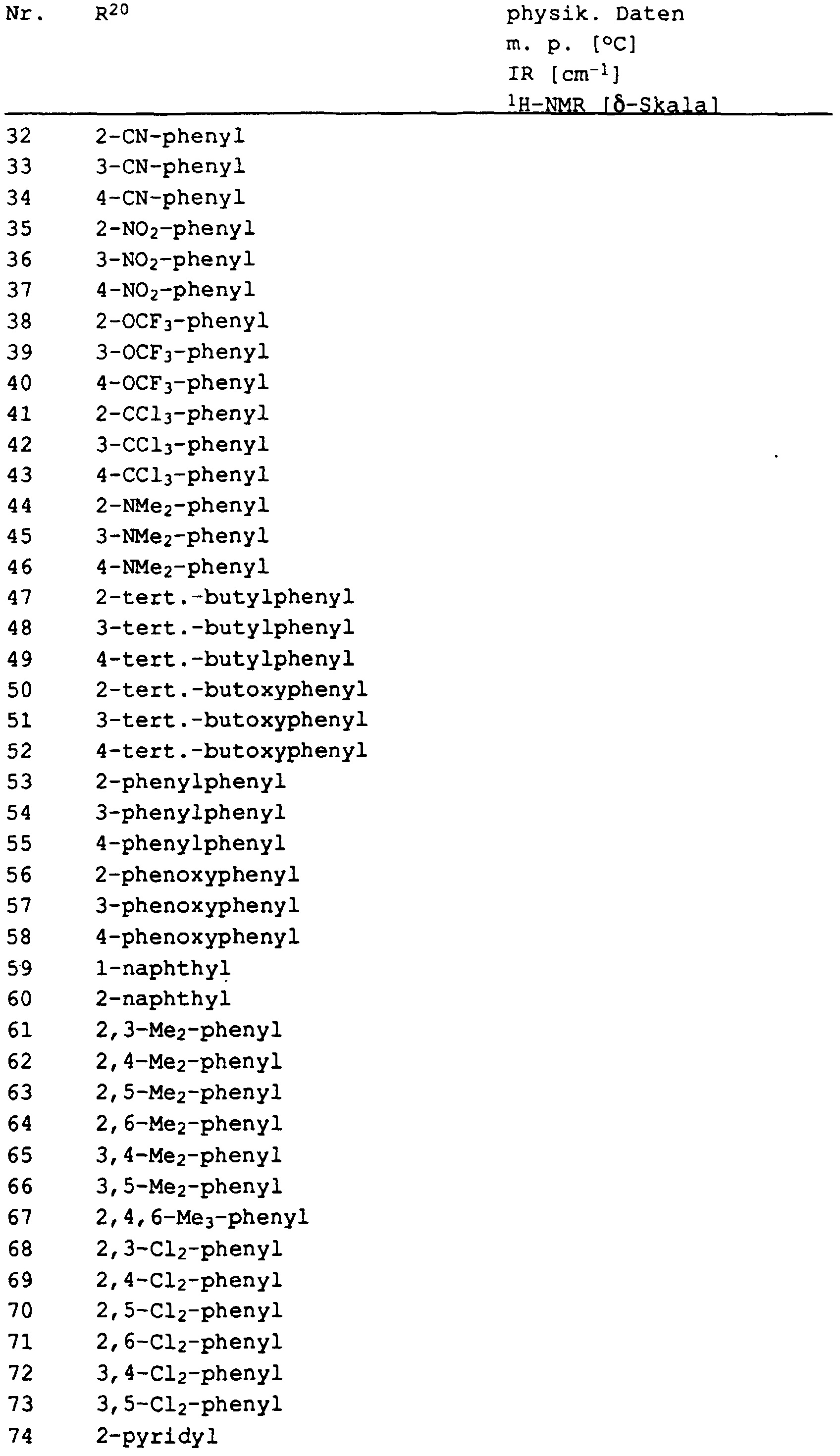

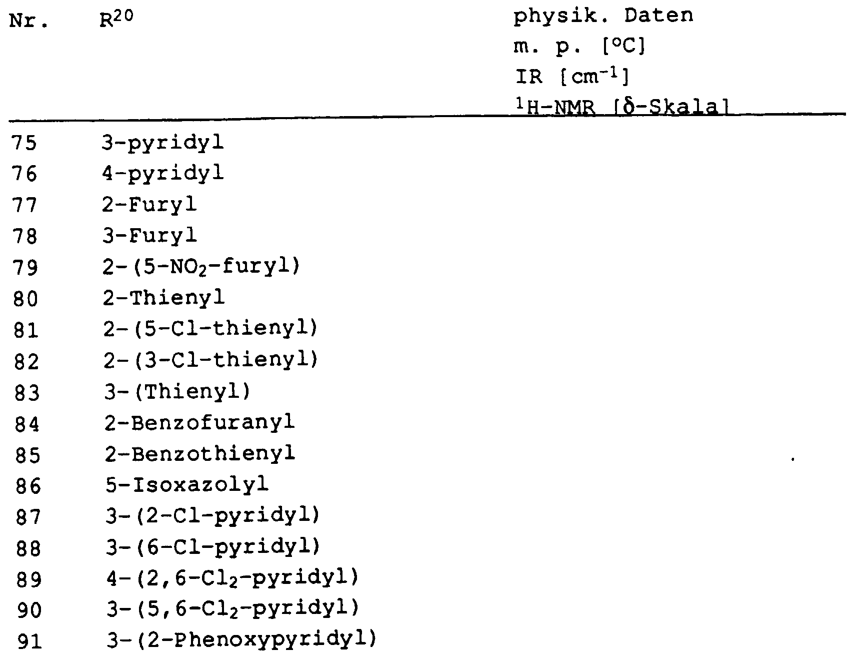

Die neuen Verbindungen eignen sich als Fungizide.

Die erfindungsgemäßen fungiziden Verbindungen bzw. die sie enthaltenden Mittel können beispielsweise in Form von direkt versprühbaren Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungformen richten sich nach den Verwendungszwecken; sie sollten in jedem Fall möglichst die feinste Verteilung der erfindungsgemäßen Wirkstoffe gewährleisten.

Normalerweise werden die Pflanzen mit den Wirkstoffen besprüht oder bestäubt oder die Samen der Pflanzen mit den Wirkstoffen behandelt.

Die Formulierungen werden in bekannter Weise hergestellt, z.B. durch Verstrecken des Wirkstoffs mit Lösungsmitteln und/oder Trägerstoffen, gewünschtenfalls unter Verwendung von Emulgiermitteln und Dispergiermitteln, wobei im Falle von Wasser als Verdünnungsmittel auch andere organische Lösungsmittel als Hilfslösungsmittel verwendet werden können. Als Hilfsstoffe kommen dafür im wesentlichen in Betracht: Lösungsmittel wie Aromaten (z.B. Xylol), chlorierte Aromaten (z.B. Chlorbenzole), Paraffine (z.B. Erdölfraktionen), Alkohole (z.B. Methanol, Butanol), Ketone (z.B. Cyclohexanon), Amine (z.B. Ethanolamin, Dimethylformamid) und Wasser; Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel wie nichtionogene und anionische Emulgatoren (z.B. Polyoxyethylen-Fettalkohol-Ether, Alkylsulfonate und Arylsulfonate) und Dispergiermittel wie Ligninsulfitablaugen und Methylcellulose.

Als oberflächenaktive Stoffe kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Laurylether- und Fettalkoholsulfaten, sowie Salze sulfatierter Hexa-, Hepta- und Octadecanolen, sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenol-, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether oder Polyoxypropylen, Laurylalkoholpolyglykoletheracetat, Sorbitester, Lignin-Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Tragerstoffe hergestellt werden. Feste Trägerstoffe sind Mineralerden wie Silicagel, Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver oder andere feste Trägerstoffe.

Beispiele für solche Zubereitungen sind:
- I.: eine Lösung aus 90 Gew.-Teilen der Verbindung Nr. 1, Tabelle 2 und 10 Gew.-Teilen N-Methyl-a-pyrrolidon, die zur Anwendung in Form kleinster Tropfen geeignet ist;
- II.: eine Mischung aus 20 Gew.-Teilen der Verbindung Nr. 24, Tabelle 2, 80 Gew.Teilen Xylol, 10 Gew.-Teilen des Anlagerungsproduktes von 8 bis 10 Mol Ethylenoxid an 1 Mol Ölsäure-N-monoethanolamid, 5 Gew.Teilen Calciumsalz der Dodecylbenzolsulfonsäure, 5 Gew.- Teilen des Anlagerungsproduktes von 40 Mol Ethylenoxid an 1 Mol Ricinusöl; durch feines Verteilen der Lösung in Wasser erhält man eine Dispersion.
- III.: eine wäßrige Dispersion aus 20 Gew.-Teilen der Verbindung Nr. 25, Tabelle 2, 40 Gew.-Teilen Cyclohexanon, 30 Gew.-Teilen Isobutanol, 20 Gew.-Teilen des Anlagerungsproduktes von 40 mol Ethylenoxid an 1 mol Ricinusöl;
- IV.: eine wäßrige Dispersion aus 20 Gew.-Teilen der Verbindung Nr. 26, Tabelle 2, 25 Gew.-Teilen Cyclohexanol, 65 Gew.-Teilen einer Mineralölfraktion vom Siedepunkt 210 bis 280'C und 10 Gew.-Teilen des Anlagerungsproduktes von 40 mol Ethylenoxid an 1 mol Ricinusöl;
- V.: eine in einer Hammermühle vermahlene Mischung aus 80 Gew.-Teilen der Verbindung Nr. 31, Tabelle 2, 3 Gew.-Teilen des Natriumsalzes der Diisobutylnaphtalin-a-sulfonsäure, 10 Gew.-Teilen des Natriumsalzes einer Ligninsulfonsäure aus einer Sulfitablauge und 7 Gew.-Teilen pulverförmigem Kieselsäuregel; durch feines Verteilen der Mischung in Wasser erhält man eine Spritzbrühe;
- VI.: eine innige Mischung aus 3 Gew.-Teilen der Verbindung Nr. 33, Tabelle 2 und 97 Gew.-Teilen feinteiligem Kaolin; dieses Stäubemittel enthält 3 Gew.-% Wirkstoff;
- VII.: eine innige Mischung aus 30 Gew.-Teilen der Verbindung Nr. 34, Tabelle 2, 92 Gew.-Teilen pulverförmigem Kieselsäuregel und 8 Gew.-Teilen Paraffinöl, das auf die Oberfläche dieses Kieselsäuregels gesprüht wurde; diese Aufbereitung gibt dem Wirkstoff eine gute Haftfähigkeit;
- VIII.: eine stabile waßrige Dispersion aus 40 Gew.-Teilen der Verbindung Nr. 35, Tabelle 2, 10 Gew.-Teilen des Natriumsalzes eines Phenolsulfonsäure-harnstoff-formaldehyd-Kondensates, 2 Gew.-Teilen Kieselgel und 48 Gew.-Teilen Wasser, die weiter verdünnt werden kann;
- IX.: eine stabile ölige Dispersion aus 20 Gew.-Teilen der Verbindung Nr. 42, Tabelle 2, 2 Gew.-Teilen des Calciumsalzes der Dodecylbenzolsulfonsäure, 8 Gew.-Teilen Fettalkohol-polyglykolether, 20 Gew.-Teilen des Natriumsalzes eines Phenolsulfonsäure-harnstoff-formaldehydKondensates und 68 Gew.-Teilen eines paraffinischen Mineralöls.

Die Wirkstoffe zeichnen sich durch eine hervorragende Wirksamkeit gegen ein breites Spektrum von pflanzenpathogenen Pilzen, insbesondere aus der Klasse der Ascomyceten und Basidiomyceten, aus. Sie sind zum Teil systemisch wirksam und können als Blatt- und Bodenfungizide eingesetzt werden.

Besondere Bedeutung haben sie für die Bekämpfung einer Vielzahl von Pilzen an verschiedenen Kulturpflanzen wie Weizen, Roggen, Gerste, Hafer, Reis, Mais, Gras, Baumwolle, Soja, Kaffee, Zuckerrohr, Wein, Obst- und Zierpflanzen und Gemüsepflanzen wie Gurken, Bohnen und Kürbisgewächsen, sowie an den Samen dieser Pflanzen.

Die Verbindungen werden angewendet, indem man die Pilze oder die vor Pilzbefall zu schützenden Saatgüter, Pflanzen, Materialien oder den Erdboden mit einer fungizid wirksamen Menge der Wirkstoffe behandelt.

Die Anwendung erfolgt vor oder nach der Infektion der Materialien, Pflanzen oder Samen durch die Pilze.

Speziell eignen sich die Verbindungen I zur Bekämpfung folgender Pflanzenkrankheiten:
Erysiphe graminis (echter Mehltau) in Getreide,
Erysiphe cichoracearum und Sphaerotheca fuliginea an Kürbisgewächsen,
Podosphaera leucotricha an Äpfeln,
Uncinula necator an Reben,
Puccinia-Arten an Getreide,
Rhizoctonia-Arten an Baumwolle und Rasen,
Ustilago-Arten an Getreide und Zuckerrohr,
Venturia inaequalis (Schorf) an Äpfeln,
Helminthosporium-Arten an Getreide,
Septoria nodorum an Weizen,
Botrytis cinerea (Grauschimmel) an Erdbeeren, Reben,
Cercospora arachidicola an Erdnüssen,
Pseudocercosporella herpotrichoides an Weizen, Gerste,
Pyricularia oryzae an Reis,
Phytophthora infestans an Kartoffeln und Tomaten,
Fusarium- und Verticillium-Arten an verschiedenen Pflanzen,
Plasmopara viticola an Reben,
Alternaria-Arten an Gemüse und Obst.

Die neuen Verbindungen können auch im Materialschutz (Holzschutz) eingesetzt werden, z.B. gegen Paecilomyces variotii.

Die fungiziden Mittel enthalten im allgemeinen zwischen 0,1 und 95, vorzugsweise zwischen 0,5 und 90 Gew.% Wirkstoff.

Die Aufwandmengen liegen je nach Art des gewünschten Effektes zwischen 0,02 und 3 kg Wirkstoff pro ha.

Bei der Saatgutbehandlung werden im allgemeinen Wirkstoffmengen von 0,001 bis 50 g, vorzugsweise 0,01 bis 10 g je Kilogramm Saatgut benötigt.

Die erfindungsgemäßen Mittel können in der Anwendungsform als Fungizide auch zusammen mit anderen Wirkstoffen vorliegen, der z.B. mit Herbiziden, Insektiziden, Wachstumsregulatoren, Fungiziden oder auch mit Düngemitteln.

Beim Vermischen mit Fungiziden erhält man dabei in vielen Fällen eine Vergrößerung des fungiziden Wirkungsspektrums.

### Anwendungsbeispiele

Als Vergleichswirkstoffe wurden die Verbindungen U, V, W = H, A = CH₃ON=, B = CH₃O und R = Phenyl (Verbindung A), R = 2-Chlorphenyl (Verbindung B), R = 4-CF₃-phenyl (Verbindung C) - alle bekannt aus EP-A 253 213 - verwendet.

### Anwendungsbeispiel 1

### Wirksamkeit gegen Gurkenmehltau

Junge Gurkenpflanzen der Sorte "Chinesische Schlange" wurden im Zweiblattstadium mit einer wäßrigen Spritzbrühe bis zur Tropfnässe besprüht. Am nächsten Tag wurden diese Pflanzen mit wäßriger Konidiensuspension des Gurkenmehltaus (Erysiphe cichoracearum und Sphaerotheca fuliginea) besprüht und im Gewächshaus bei Temperaturen zwischen 20 und 22°C und 70 bis 80% Luftfeuchtigkeit aufgestellt. 21 Tage nach der Wirkstoffapplikation wurde das Ausmaß des Pilzbefalls ermittelt.

Das Ergebnis zeigt, daß die Wirkstoffe aus Tabelle 2, Nr. 148 (2,148) und 2,166 bei der Anwendung als 63 ppm Wirkstoff enthaltende Spritzbrühe eine bessere fungizide Wirkung zeigen (10% Befall der Blätter) als die bekannten Vergleichswirkstoffe A, B und C (45% Befall der Blätter).

### Anwendungsbeispiel 2

### Wirksamkeit gegen Botrytis cinerea an Paprikaschoten (Fruchttest)

Scheiben von grünen Paprikaschoten wurden mit wäßriger Wirkstoffaufbereitung, die 80% Wirkstoff und 20% Emulgiermittel in der Trockensubstanz enthielt, tropfnaß besprüht. 2 Stunden nach dem Antrocknen des Spritzbelags wurden die Fruchtscheiben mit einer Sporensuspension von Botrytis cinerea, die 1,7×10⁶ Sporen pro ml einer 2%-igen Biomalzlösung enthielt, inokuliert. Die inokulierten Fruchtscheiben wurden anschließend in feuchten Kammern bei 18°C für 4 Tage inkubiert. Dann erfolgte visuell die Auswertung der Botrytis-Entwicklung auf den befallenen Fruchtscheiben.

Das Ergebnis zeigt, daß die Wirkstoffe 2,25; 2,166 und 2,179 bei der Anwendung als 500 ppm Wirkstoff enthaltende Spritzbrühe eine bessere fungizide Wirkung zeigen (10% Befall der Früchte) als der bekannte Vergleichswirkstoff A (30% Befall der Früchte).

### Anwendungsbeispiel 3

### Wirksamkeit gegen Plasmopara viticola

Blätter von Topfreben der Sorte "Müller Thurgau" wurden mit wäßriger Spritzbrühe, die 80% Wirkstoff und 20% Emulgiermittel in der Trockensubstanz enthielt, besprüht. Um die Wirkungsdauer der Wirkstoffe beurteilen zu können, wurden die Pflanzen nach dem Antrocknen des Spritzbelags 8 Tage im Gewächshaus aufgestellt. Erst dann wurden die Blätter mit einer Zoosporenaufschwemmung von Plasmopara viticola (Rebenperonospora) infiziert. Danach wurden die Reben zunächst für 48 Stunden in einer wasserdampfgesättigten Kammer bei 24°C und anschließend für 5 Tage in einem Gewächshaus bei Temperaturen zwischen 20 und 30°C aufgestellt. Nach dieser Zeit wurden die Pflanzen zur Beschleunigung des Sporangienträgerausbruchs abermals für 16 Stunden in der feuchten Kammer aufgestellt. Dann erfolgte die Beurteilung des Ausmaßes des Pilzausbruchs auf den Blattunterseiten.

Das Ergebnis zeigt, daß die Wirkstoffe 2,25; 2,92; 2,94; 2,95 und 2,148 bei der Anwendung als 16 ppm Wirkstoff enthaltende Spritzbrühe eine bessere fungizide Wirkung zeigen (5% Befall der Blätter) als der bekannte Vergleichswirkstoff C (35% Befall der Früchte).

## Patentansprüche

1. Acetylenderivate der allgemeinen Formel I 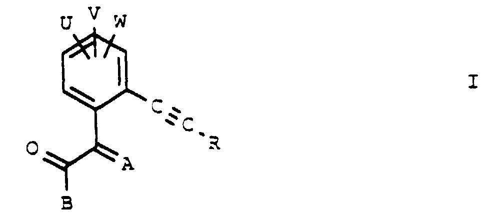 in der
U,V,W gleich oder verschieden sind und Wasserstoff, Halogen, Nitro, Cyano, C₁-C₄-Alkyl und C₁-C₄-Alkoxy bedeuten,
a) A C₁-C₄-Alkoxyimino oder C₁-C₄-Alkyliden und B C₁-C₄-Alkoxy bedeutet und
R Wasserstoff, Halogen, Cyano, CF₃, ggf. verzweigtes C₁-C₁₀-Alkyl, Halogen-C₁-C₄-alkyl, C₁-C₄-Alkoxy-C₁-C₄-alkyl, C₂-C₆-Alkenyl, C₂-C₄-Alkinyl, C₃-C₆-Cycloalkyl, ggf. subst. Heterocyclyl, ggf. subst. Heteroaryl, ggf. subst. Aryl-C₁-C₄-alkyl, ggf. subst. Aryloxy-C₁-C₄-alkyl, ggf. subst. Arylamino-C₁-C₄-alkyl, ggf. subst. Arylthiomethyl, ggf. subst. Aryl-C₂-C₄-alkenyl, ggf. subst. Arylethinyl, ggf. subst. Heteroaryl-C₁-C₄-alkyl, ggf. subst. Heteroaryloxy-C₁-C₄-alkyl oder ggf. subst. Heteroarylthiomethyl bedeutet,
COR¹, CONR²R³, CSNR⁴R⁵, COSR⁶, CSOR⁷, CSSR⁸, CH(OH)R⁹, CH(OR¹⁰)R¹¹, SiR¹²R¹³R¹⁴, SnR¹⁵R¹⁶R¹⁷, COR¹⁸ oder CR²⁰=NOR¹⁹ bedeutet, oder
Aryl bedeutet, welches einen der folgenden Substituenten trägt: Cyano, C₁-C₄-Halogenalkoxy, C₁-C₄-Alkoxyimino-C₁-C₄-alkyl, Aryl, Aryloxy, Benzyl, Benzyloxy, Heteroaryl, Heteroaryloxy, C₃-C₆-Cycloalkyl, C₁-C₄-Dialkylamino, CO₂CH₃, CO₂C₂H₅, Formyl und Acyl, wobei der Ausdruck Heteroaryl einen aromatischen Fünfring- oder Sechsringheterocyclus bedeutet, oder
Aryl bedeutet, welches mindestens zwei Substituenten aus der folgenden Gruppe trägt: Halogen, Cyano, Nitro, C₁-C₄-Alkyl, C₁-C₄-Alkoxy, C₁-C₄-Halogenalkyl, C₁-C₄-Halogenalkoxy, C₁-C₄-Alkoxyimino-C₁-C₄-alkyl, Aryl, Aryloxy, Benzyl, Benzyloxy, Heteroaryl, Heteroaryloxy, C₃-C₆-Cycloalkyl, C₁-C₄-Dialkylamino, CO₂CH₃, CO₂C₂H₅, Formyl und Acyl, wobei der Ausdruck Heteroaryl einen aromatischen Fünfring- oder Sechsringheterocyclus bedeutet, oder
b) A C₁-C₄-Alkoxyimino, C₁-C₄-Alkylthiomethyliden oder C₁-C₄-Alkoxymethyliden und B C₁-C₄-Alkylamino und
R Wasserstoff, Halogen, Cyano, CF₃, Halogen-C₁-C₄-alkyl, C₁-C₄-Alkoxy-C₁-C₄-alkyl, C₂-C₆-Alkenyl, C₂-C₄-Alkinyl, C₃-C₆-Cycloalkyl, ggf. subst. Heterocyclyl, ggf. subst. Heteroaryl, ggf. subst. Aryl-C₁-C₄-alkyl, ggf. subst. Aryloxy-C₁-C₄-alkyl, ggf. subst. Arylamino-C₁-C₄-alkyl, ggf. subst. Arylthiomethyl, ggf. subst. Aryl-C₂-C₄-alkenyl, ggf. subst. Arylethinyl, ggf. subst. Heteroaryl-C₁-C₄-alkyl, ggf. subst. Heteroaryloxy-C₁-C₄-alkyl oder ggf. subst. Heteroarylthiomethyl bedeutet,
COR¹, CONR²R³, CSNR⁴R⁵, COSR⁶, CSOR⁷, CSSR⁸, CH(OH)R⁹, CH(OR¹⁰)R¹¹, SiR¹²R¹³R¹⁴, SnR¹⁵R¹⁶R¹⁷, COR¹⁸ oder CR²⁰=NOR¹⁹ bedeutet, oder
Aryl bedeutet, welches mindestens einen Substituenten aus der folgenden Gruppe trägt: Halogen, Cyano, Nitro, C₁-C₄-Alkyl, C₁-C₄-Alkoxy, C₁-C₄-Halogenalkyl, C₁-C₄-Halogenalkoxy, C₁-C₄-Alkoxyimino-C₁-C₄-alkyl, Aryl, Aryloxy, Benzyl, Benzyloxy, Heteroaryl, Heteroaryloxy, C₃-C₆-Cycloalkyl, C₁-C₄-Dialkylamino, CO₂CH₃, CO₂C₂H₅, Formyl und Acyl, wobei der Ausdruck Heteroaryl einen aromatischen Fünfring- oder Sechsringheterocyclus bedeutet, oder
c) A C₁-C₄-Alkyliden und B C₁-C₄-Alkylamino oder
A C₁-C₄-Alkylthiomethyliden und B C₁-C₄-Alkoxy oder
A C₁-C₄-Alkyliden, C₁-C₄-Alkoxymethyliden, C₁-C₄-Alkylthiomethyliden und C₁-C₄-Alkoxyimino und B Hydroxy bedeutet und
R Wasserstoff, Halogen, Cyano, CF₃, ggf. verzweigtes C₁-C₁₀-Alkyl, Halogen-C₁-C₄-alkyl, C₁-C₄-Alkoxy-C₁-C₄-alkyl, C₂-C₆-Alkenyl, C₂-C₄-Alkinyl, C₃-C₆-Cycloalkyl, ggf. subst. Heterocyclyl, ggf. subst. Aryl, ggf. subst. Heteroaryl, ggf. subst. Aryl-C₁-C₄-alkyl, ggf. subst. Aryloxy-C₁-C₄-alkyl, ggf. subst. Arylamino-C₁-C₄-alkyl, ggf. subst. Arylthiomethyl, ggf. subst. Aryl-C₂-C₄-alkenyl, ggf. subst. Arylethinyl, ggf. subst. Heteroaryl-C₁-C₄-alkyl, ggf. subst. Heteroaryloxy-C₁-C₄-alkyl oder ggf. subst. Heteroarylthiomethyl bedeutet,
COR¹, CONR²R³, CSNR⁴R⁵, COSR⁶, CSOR⁷, CSSR⁸, CH(OH)R⁹, CH(OR¹⁰)R¹¹, SiR¹²R¹³R¹⁴, SnR¹⁵R¹⁶R¹⁷, COR¹⁸ oder CR²⁰=NOR¹⁹ bedeutet,
R¹-R¹⁷, R¹⁹ und R²⁰ gleich oder verschieden sind und
Wasserstoff, C₁-C₄-Alkyl, ggf. subst. Aryl, ggf. subst. Heteroaryl, ggf. subst. Arylalkyl und ggf. subst. Heteroarylalkyl bedeuten,
R¹⁸ OH, C₁-C₄-Alkoxy und ggf. subst. Aryl-C₁-C₄-alkoxy bedeutet wobei der Ausdruck "ggf. subst." neben Wasserstoff für die Substituenten Halogen, Cyano, Nitro, C₁-C₄-Alkyl, C₁-C₄-Alkoxy, C₁-C₄-Halogenalkyl, C₁-C₄-Halogenalkoxy, C₁-C₄-Alkoxyimino-C₁-C₄-alkyl, Aryl, Aryloxy, Benzyl, Benzyloxy, Heteroaryl, Heteroaryloxy, C₃-C₆-Cycloalkyl, C₁-C₄-Dialkylamino, CO₂CH₃, CO₂C₂H₅, Formyl und Acyl steht und der Ausdruck Heteroaryl einen aromatischen Fünfring- oder Sechsringheterocyclus bedeutet.

2. Acetylenderivate der Formel I gemäß Anspruch I, in denen U, V und W Wasserstoff bedeuten, A für Methoxyimino und B für Methoxy steht.

3. Acetylenderivate der Formel I gemäß Anspruch I, in denen U, V und W Wasserstoff bedeuten, A für Ethyliden und B für Methoxy steht.

4. Acetylenderivate der Formel I gemäß Anspruch I, in denen U, V und W Wasserstoff bedeuten, A für Methoxyimino und B für Methylamino steht.

5. Acetylenderivate der Formel I gemäß Anspruch I, in denen U, V und W Wasserstoff bedeuten, A für Methyl-thiomethyliden und B für Methoxy steht.

6. Acetylenderivate der Formel I gemäß Anspruch I, in denen U, V, W und R für Wasserstoff stehen.

7. α-Ketoester der Formel VIII 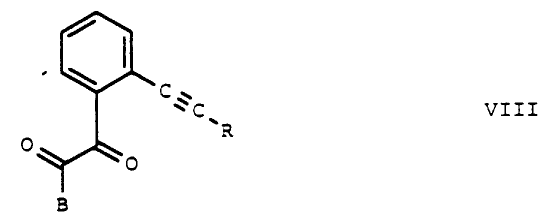 in der R und B die in Anspruch 1 gegebene Bedeutung haben.

8. Fungizid, enthaltend einen inerten Trägerstoff und eine fungizid wirksame Menge eines Acetylenderivats der Formel I gemäß Anspruch 1.

9. Verfahren zur Bekämpfung von Pilzen dadurch gekennzeichnet, daß man die Pilze oder die von Pilzbefall bedrohten Materialien, Pflanzen, Saatgut oder den Erdboden mit einer fungizid wirksamen Menge eines Acetylenderivats der Formel I gemäß Anspruch 1 behandelt.

10. verfahren zur Bekämpfung von Insekten, Nematoden und Spinnmilben, dadurch gekennzeichnet, daß man diese oder den Ort, an dem sie sich aufhalten, mit einer wirksamen Menge eines Acetylenderivats der Formel I gemäß Anspruch 1 behandelt.

## Claims

1. An acetylene derivative of the formula I 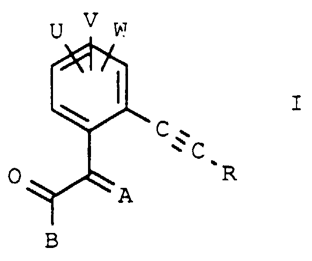 where
U, V and W are identical or different and are each hydrogen, halogen, nitro, cyano, C₁-C₄-alkyl or C₁-C₄-alkoxy,
a) A is C₁-C₄-alkoxyimino or C₁-C₄-alkylidene and B C₁-C₄-alkoxy and
R is hydrogen, halogen, cyano, CF₃, straight-chain or branched C₁-C₁₀-alkyl, halo-C₁-C₄-alkyl, C₁-C₄-alkoxy-C₁-C₄-alkyl, C₂-C₆-alkenyl, C₂-C₄-alkinyl, C₃-C₆-cycloalkyl, unsubstituted or substituted heterocyclyl, unsubstituted or substituted hetaryl, unsubstituted or substituted aryl-C₁-C₄-alkyl, unsubstituted or substituted aryloxy-C₁-C₄-alkyl, unsubstituted or substituted arylamino-C₁-C₄-alkyl, unsubstituted or substituted arylthiomethyl, unsubstituted or substituted aryl-C₂-C₄-alkenyl, unsubstituted or substituted arylethinyl, unsubstituted or substituted hetaryl-C₁-C₄-alkyl, unsubstituted or substituted hetaryloxy-C₁-C₄-alkyl or unsubstituted or substituted hetarylthiomethyl,
COR¹, CONR²R³, CSNR⁴R⁵, COSR⁶, CSOR⁷, CSSR⁸, CH(OH)R⁹, CH(OR¹⁰)R¹¹, SiR¹²R¹³R¹⁴, SnR¹⁵R¹⁶R¹⁷, COR¹⁸ or CR²⁰=NOR¹⁹, or
aryl which carries one of the following substituents: cyano, C₁-C₄-haloalkoxy, C₁-C₄-alkoxyimino-C₁-C₄-alkyl, aryl, aryloxy, benzyl, benzyloxy, hetaryl, hetaryloxy, C₃-C₆-cycloalkyl, C₁-C₄-dialkylamino, CO₂CH₃, CO₂C₂H₅, formyl and acyl, the expression hetaryl being an aromatic five-membered or six-membered heterocyclic structure, or
aryl which carries at least two substituents from the following group: halogen, cyano, nitro, C₁-C₄-alkyl, C₁-C₄-alkoxy, C₁-C₄-haloalkyl, C₁-C₄-haloalkoxy, C₁-C₄-alkoxyimino-C₁-C₄-alkyl, aryl, aryloxy, benzyl, benzyloxy, hetaryl, hetaryloxy, C₃-C₆-cycloalkyl, C₁-C₄-dialkylamino, CO₂CH₃, CO₂C₂H₅, formyl and acyl, the expression hetaryl being an aromatic five-membered or six-membered heterocyclic structure, or
b) A is C₁-C₄-alkoxyimino, C₁-C₄-alkylthiomethylidene or C₁-C₄-alkoxymethylidene and B is C₁-C₄-Alkylamino and
R is hydrogen, halogen, cyano, CF₃, halo-C₁-C₄-alkyl, C₁-C₄-alkoxy-C₁-C₄-alkyl, C₂-C₆-alkenyl, C₂-C₄-alkinyl, C₃-C₆-cycloalkyl, unsubstituted or substituted heterocyclyl, unsubstituted or substituted hetaryl, unsubstituted or substituted aryl-C₁-C₄-alkyl, unsubstituted or substituted aryloxy-C₁-C₄-alkyl, unsubstituted or substituted arylamino-C₁-C₄-alkyl, unsubstituted or substituted arylthiomethyl, unsubstituted or substituted aryl-C₂-C₄-alkenyl, unsubstituted or substituted arylethinyl, unsubstituted or substituted hetaryl-C₁-C₄-alkyl, unsubstituted or substituted hetaryloxy-C₁-C₄-alkyl or unsubstituted or substituted hetarylthiomethyl,
COR¹, CONR²R³, CSNR⁴R⁵, COSR⁶, CSOR⁷, CSSR⁸, CH(OH)R⁹, CH(OR¹⁰)R¹¹, SiR¹²R¹³R¹⁴, SnR¹⁵R¹⁶R¹⁷, COR¹⁸ or CR²⁰=NOR¹⁹, or
aryl which carries at least one substituent from the following group: halogen, cyano, nitro, C₁-C₄-alkyl, C₁-C₄-alkoxy, C₁-C₄-haloalkyl, C₁-C₄-haloalkoxy, C₁-C₄-alkoxyimino-C₁-C₄-alkyl, aryl, aryloxy, benzyl, benzyloxy, hetaryl, hetaryloxy, C₃-C₆-cycloalkyl, C₁-C₄-dialkylamino, CO₂CH₃, CO₂C₂H₅, formyl and acyl, the expression hetaryl being an aromatic five-membered or six-membered heterocyclic structure, or
c) A is C₁-C₄-alkylidene and B is C₁-C₄-alkylamino or A is C₁-C₄-alkylthiomethylidene and B is C₁-C₄-alkoxy or A is C₁-C₄-alkylidene, C₁-C₄-alkoxymethylidene, C₁-C₄-alkylthiomethylidene and C₁-C₄-alkoxyimino and B is hydroxyl and
R is hydrogen, halogen, cyano, CF₃, straight-chain or branched C₁-C₁₀-alkyl, halo-C₁-C₄-alkyl, C₁-C₄-alkoxy-C₁-C₄-alkyl, C₂-C₆-alkenyl, C₂-C₄-alkinyl, C₃-C₆-cycloalkyl, unsubstituted or substituted heterocyclyl, unsubstituted or substituted aryl, unsubstituted or substituted hetaryl, unsubstituted or substituted aryl-C₁-C₄-alkyl, unsubstituted or substituted aryloxy-C₁-C₄-alkyl, unsubstituted or substituted arylamino-C₁-C₄-alkyl, unsubstituted or substituted arylthiomethyl, unsubstituted or substituted aryl-C₂-C₄-alkenyl, unsubstituted or substituted arylethinyl, unsubstituted or substituted hetaryl-C₁-C₄-alkyl, unsubstituted or substituted hetaryloxy-C₁-C₄-alkyl or unsubstituted or substituted hetarylthiomethyl,
COR¹, CONR²R³, CSNR⁴R⁵, COSR⁶, CSOR⁷, CSSR⁸, CH(OH)R⁹, CH(OR¹⁰)R¹¹, SiR¹²R¹³R¹⁴, SnR¹⁵R¹⁶R¹⁷, COR¹⁸ or CR²⁰=NOR¹⁹,
R¹-R¹⁷, R¹⁹ and R²⁰ are identical or different and are each hydrogen, C₁-C₄-alkyl, unsubstituted or substituted aryl, unsubstituted or substituted hetaryl, unsubstituted or substituted arylalkyl or unsubstituted or substituted hetarylalkyl, and
R¹⁸ is OH, C₁-C₄-alkoxy and unsubstituted or substituted aryl-C₁-C₄-alkoxy, suitable substituents being halogen, cyano, nitro, C₁-C₄- alkyl, C₁-C₄-alkoxy, C₁-C₄-haloalkyl, C₁-C₄-haloalkoxy, C₁-C₄-alkoxyimino-C₁-C₄-alkyl, aryl, aryloxy, benzyl, benzyloxy, hetaryl, hetaryloxy, C₃-C₆-cycloalkyl, C₁-C₄-dialkylamino, CO₂CH₃, CO₂C₂H₅, formyl and acyl, and the expression hetaryl being an aromatic five-membered or six-membered heterocyclic structure.

2. An acetylene derivative of the formula I as claimed in claim 1, wherein U, V and W are each hydrogen, A is methoxyimino and B is methoxy.

3. An acetylene derivative of the formula I as claimed in claim 1, wherein U, V and W are each hydrogen, A is ethylidene and B is methoxy.

4. An acetylene derivative of the formula I as claimed in claim 1, wherein U, V and W are each hydrogen, A is methoxyimino and B is methylamino.

5. An acetylene derivative of the formula I as claimed in claim 1, wherein U, V and W are each hydrogen, A is methylthiomethylidene and B is methoxy.

6. An acetylene derivative of the formula I as claimed in claim 1, wherein U, V, W and R are each hydrogen.

7. An α-ketoester of the formula VIII 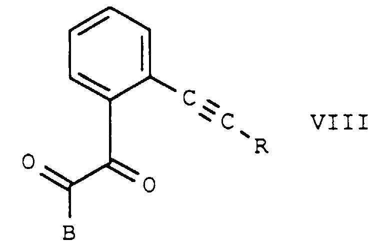 where R and B have the meanings given in claim 1.

8. A fungicide containing an inert carrier and a fungicidal amount of an acetylene derivative of the formula I as claimed in claim 1.

9. A method for controlling fungi, wherein the fungi or the materials, plants or seed threatened by fungal attack or the soil is or are treated with a fungicidal amount of an acetylene derivative of the formula I as claimed in claim 1.

10. A method for controlling insects, nematodes and spider mites, wherein these or the place where they are present is or are treated with an effective amount of an acetylene derivative of the formula I as claimed in claim 1.

## Revendications

1. Dérivés acétyléniques de formule générale I 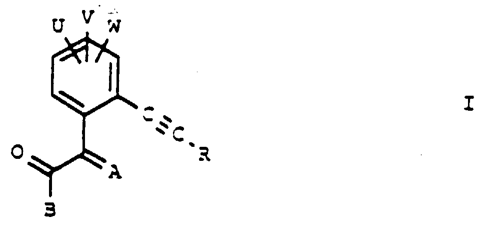 dans laquelle
U, V, W ayant des significations identiques ou différentes représentent chacun l'hydrogène, un halogène, un groupe nitro, cyano, alkyle en C1-C4 ou alcoxy en C1-C4,
a) A représente un groupe alcoxyimino en C1-C4 ou alkylidène en C1-C4 et B représente un groupe alcoxy en C1-C4 et
R représente l'hydrogène, un halogène, un groupe cyano, CF₃, alkyle en C1-C10 éventuellement ramifié, halogénoalkyle en C1-C4, (alcoxy en C1-C4)alkyle en C1-C4, alcényle en C2-C6, alcynyle en C2-C4, cycloalkyle en C3-C6, hétérocycle éventuellement substitué, hétéroaryle éventuellement substitué, aryl-alkyle en C1-C4 éventuellement substitué, aryloxy-alkyle en C1-C4 éventuellement substitué, arylamino-alkyle en C1-C4 éventuellement substitué, arylthiométhyle éventuellement substitué, aryl-alcényle en C2-C4 éventuellement substitué, aryléthynyle éventuellement substitué, hétéroaryl-alkyle en C1-C4 éventuellement substitué, hétéroaryloxy-alkyle en C1-C4 éventuellement substitué ou hétéroarylthiométhyle éventuellement substitué,
COR¹, CONR²R³, CSNR⁴R⁵, COSR⁶, CSOR⁷, CSSR⁸, CH(OH)R⁹, CH(OR¹⁰)R¹¹, SiR¹²R¹³R¹⁴, SnR¹⁵R¹⁶R¹⁷, COR¹⁸ ou CR²⁰ = NOR¹⁹
ou bien
un groupe aryle portant l'un des substituants suivants : cyano, halogénoalcoxy en C1-C4, (alcoxy en C1-C4)-imino-alkyle en C1-C4, aryle, aryloxy, benzyle, benzyloxy, hétéroaryle, hétéroaryle, cycloalkyle en C3-C6, di-(alkyle en C1-C4)amino, CO₂CH₃, CO₂C₂H₅, formyle ou acyle, l'expression hétéroaryle désignant un hétérocycle aromatique à cinqu ou six chaînons, ou bien
un groupe aryle portant au moins deux substituants du groupe suivant : halogéno, cyano, nitro, alkyle en C1-C4, alcoxy en C1-C4, halogénoalkyle en C1-C4, halogénoalcoxy en C1-C4 (alcoxy en C1-C4)imino-alkyle en C1-C4, aryle, aryloxy, benzyle, benzyloxy, hétéroaryle, hétéroaryloxy, cycloalkyle en C3-C6, di-(alkyle en C1-C4)amino, CO₂CH₃, CO₂C₂H₅, formyle et acyle, l'expression hétéroaryle désignant un hétérocycle aromatique à cinq ou six chaînons, ou bien
b) A représente un groupe alcoxyimino en C1-C4, (alkyle en C1-C4)thiométhylidène ou (alcoxy en C1-C4)méthylidène et B représente un groupe alkylamino en C1-C4 et
R représente l'hydrogène, un halogène, un groupe cyano, CF₃, halogénoalkyle en C1-C4, (alcoxy en C1-C4)alkyle en C1-C4, alcényle en C2-C6, alcynyle en C2-C4, cycloalkyle en C3-C6, hétérocycle éventuellement substitué, hétéroaryle éventuellement substitué, aryl-alkyle en C1-C4 éventuellement substitué, aryloxy-alkyle en C1-C4 éventuellement substitué, arylamino-alkyle en C1-C4 éventuellement substitué, arylthiométhyle éventuellement substitué, aryl-alcényle en C2-C4 éventuellement substitué, aryléthynyle éventuellement substitué, hétéroaryl-alkyle en C1-C4 éventuellement substitué, hétéroaryloxy-alkyle en C1-C4 éventuellement substitué ou hétéroarylthiométhyle éventuellement substitué,
COR¹, CONR²R³, CSNR⁴R⁵, COSR⁶, CSOR⁷, CSSR⁸, CH(OH)R⁹, CH(OR¹⁰)R¹¹, SiR¹²R¹³R¹⁴, SnR¹⁵R¹⁶R¹⁷, COR¹⁸ ou CR²⁰=NOR¹⁹
ou bien
un groupe aryle portant au moins un substituant du groupe suivant : halogéno, cyano, nitro, alkyle en C1-C4, alcoxy en C1-C4, halogénoalkyle en C1-C4, halogénoalcoxy en C1-C4, (alcoxy en C1-C4)imino-alkyle en C1-C4, aryle, aryloxy, benzyle, benzyloxy, hétéroaryle, hétéroaryloxy, cycloalkyle en C3-C6, di-(alkyle en C1-C4)amino, CO₂CH₃, CO₂C₂H₅, formyle et acyle, l'expression hétéroaryle désignant un hétérocycle aromatique à cinq ou six chaînons, ou bien
c) A représente un groupe alkylidène en C1-C4 et B un groupe alkylamino en C1-C4, ou bien
A représente un groupe (alkyle en C1-C4)thiométhylidène et B un groupe alcoxy en C1-C4, ou bien
A représente un groupe alkylidène en C1-C4, (alcoxy en C1-C4)méthylidène, (alkyle en C1-C4)thiométhylidène et (alcoxy en C1-C4)imino et B un groupe hydroxy, et
R représente l'hydrogène, un halogène, un groupe cyano, CF₃, alkyle en C1-C10 éventuellement ramifié, halogénoalkyle en C1-C4, (alcoxy en C1-C4)alkyle en C1-C4, alcényle en C2-C6, alcynyle en C2-C4, cycloalkyle en C3-C6, hétérocycle éventuellement substitué, aryle éventuellement substitué, hétéroaryle éventuellement substitué, arylalkyle en C1-C4 éventuellement substitué, aryloxy-alkyle en C1-C4 éventuellement substitué, arylamino-alkyle en C1-C4 éventuellement substitué, arylthiométhyle éventuellement substitué, aryl-alcényle en C2-C4 éventuellement substitué, hétéroaryl-alkyle en C1-C4 éventuellement substitué, hétéroaryloxy-alkyle en C1-C4 éventuellement substitué ou hétéroarylthiométhyle éventuellement substitué,
COR¹, CONR²R³, CSNR⁴R⁵, COSR⁶, CSOR⁷, CSSR⁸, CH(OH)R⁹, CH(OR¹⁰)R¹¹, SiR¹²R¹³R¹⁴, SnR¹⁵R¹⁶R¹⁷, COR¹⁸ ou CR²⁰=NOR¹⁹
R¹ à R¹⁷, R¹⁹ et R²⁰, ayant des significations identiques ou différentes, représentent chacun l'hydrogène, un groupe alkyle en C1-C4, aryle éventuellement substitué, hétéroaryle éventuellement substitué, arylalkyle éventuellement substitué ou hétéroarylalkyle éventuellement substitué,
R¹⁸ représente OH, un groupe alcoxy en C1-C4 ou un groupe aryl-alcoxy en C1-C4 éventuellement substitué,
l'expression "éventuellement substitué" se rapportant, à côté de l'hydrogène, aux substituants halogéno, cyano, nitro, alkyle en C1-C4, alcoxy en C1-C4, halogénoalkyle en C1-C4, halogénoalcoxy en C1-C4, (alcoxy en C1-C4)imino-alkyle en C1-C4, aryle, aryloxy, benzyle, benzyloxy, hétéroaryle, hétéroaryloxy, cycloalkyle en C3-C6, di(alkyle en C1-C4)amino, CO₂CH₃, CO₂C₂H₅, formyle et acyle, et l'expression hétéroaryle désignant un hétérocycle aromatique à cinq ou six chaînons.

2. Dérivés acétyléniques de formule I de la revendication 1, pour lesquels U, V et W représentent l'hydrogène, A le groupe méthoxyimino et B le groupe méthoxy.

3. Dérivés acétyléniques de formule I de la revendication 1, pour lesquels U, V et W représentant l'hydrogène , A le groupe éthylidène et B le groupe méthoxy.

4. Dérivés acétyléniques de formule I de la revendication 1, pour lesquels U, V et W représentent l'hydrogène, A un groupe méthoxyimino et B le groupe méthylamino.

5. Dérivés acétyléniques de formule I de la revendication 1, pour lesquels U, V et W représentent l'hydrogène, A le groupe méthylthiométhylidène et B le groupe méthoxy.

6. Dérivés acétyléniques de formule I de la revendication 1, pour lesquels U, V, W et R représentent l'hydrogène.

7. α-cétoesters de formule VIII 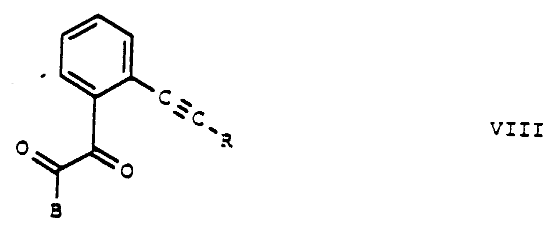 dans laquelle R et B ont les significations indiquées dans la revendication 1.

8. Produit fongicide contenant un véhicule inerte et une quantité fongicide efficace d'un dérivé acétylénique de formule I de la revendication 1.

9. Procédé pour combattre les mycètes, caractérisé par le fait que l'on traite les mycètes ou les matériaux, végétaux, semences ou sols menacés d'une attaque par les mycètes, par une quantité fongicide efficace d'un dérivé acétylénique de formule I de la revendication 1.

10. Procédé pour combattre les insectes, les nématodes et les acariens, caractérisé par le fait que l'on traite ces parasites ou les lieux qu'ils infestent par une quantité efficace d'un dérivé acétylénique de formule I de la revendication 1.
